# EUROPEAN PATENT SPECIFICATION

(11) **EP 3 740 070 B1**
(45) Date of publication and mention of the grant of the patent: **16.10.2024**
(21) Application number: 19704087.6
(22) Date of filing: 11.01.2019
(51) Int. Cl.: A01N 25/26, A01N 63/30, A01P 7/00

(54) **ALGINATE ENCAPSULATION OF FUNGAL MICROSCLEROTIA**
ALGINAT-VERKAPSELUNG FÜR PILZLICHE MIKROSCLEROTIEN
ENCAPSULATION AVEC D'ALGINATE DE MICROSCLEROTIA FONGIQUE

(30) Priority: 18.01.2018 US 201862618943 P
(43) Date of publication of application: 25.11.2020
(73) Proprietor: Pioneer Hi-Bred International Inc., Johnston, IA 50131-1014 (US)
(72) Inventor: HALLAHAN, David L., Johnston, Iowa 50131-0552 (US)
(74) Representative: J A Kemp LLP
(86) International application number: PCT/US2019/013138
(87) International publication number: WO 2019/143526

(56) References cited:
- WO-A1-2017/066094
- WO-A2-2009/035925
- FARIZ ADZMI ET AL: "Preparation, characterisation and viability of encapsulated Trichoderma harzianum UPM40 in alginate-montmorillonite clay", JOURNAL OF MICROENCAPSULATION., vol. 29, no. 3, 7 February 2012 (2012-02-07), GB, pages 205 - 210, XP055564964, ISSN: 0265-2048, DOI: 10.3109/02652048.2012.659286
- P. A. SHAH ET AL: "Method To Immobilize the Aphid-Pathogenic Fungus Erynia neoaphidis in an Alginate Matrix for Biocontrol", APPLIED AND ENVIRONMENTAL MICROBIOLOGY, 1 November 1998 (1998-11-01), United States, pages 4260 - 4263, XP055565354, Retrieved from the Internet <URL:https://aem.asm.org/content/aem/64/11/4260.full.pdf>
- J A LEWIS ET AL: "Characteristics of alginate pellets formulated with Trichoderma and Gliocladium and their effect on the proliferation of the fungi in soil", PLANT PATHOLOGY, vol. 34, 1 January 1985 (1985-01-01), pages 571 - 577, XP055565176, DOI: 10.1111/j.1365-3059.1985.tb01409.x
- ROBERTO M. PEREIRA ET AL: "Alginate and Cornstarch Mycelial Formulations of Entomopathogenic Fungi, Beauveria bassiana and Metarhizium anisopliae", JOURNAL OF ECONOMIC ENTOMOLOGY, vol. 84, no. 6, 1 December 1991 (1991-12-01), LANDHAM,MD,US, pages 1657 - 1661, XP055565072, ISSN: 0022-0493, DOI: 10.1093/jee/84.6.1657
- VIVIEN KRELL ET AL: "Encapsulation of Metarhizium brunneum enhances endophytism in tomato plants", BIOLOGICAL CONTROL, vol. 116, 11 May 2017 (2017-05-11), US, pages 62 - 73, XP055565387, ISSN: 1049-9644, DOI: 10.1016/j.biocontrol.2017.05.004
- DATABASE WPI Week 201235, Derwent World Patents Index; AN 2012-D89003, XP002789488
- DATABASE WPI Week 200961, Derwent World Patents Index; AN 2009-M79933, XP002789489
- DATABASE WPI Week 201635, Derwent World Patents Index; AN 2016-239625, XP002789490
- STEFAN T. JARONSKI ET AL: "Efficacy of Metarhizium anisopliae microsclerotial granules", BIOCONTROL SCIENCE AND TECHNOLOGY., vol. 18, no. 8, 25 September 2008 (2008-09-25), GB, pages 849 - 863, XP055565724, ISSN: 0958-3157, DOI: 10.1080/09583150802381144

## Description

### FIELD

Entomopathogenic fungal strains, entomopathogenic fungal compositions, entomopathogenic fungal propagules, and methods of processing to compositions stably encapsulating entomopathogenic fungal propagules.

### BACKGROUND

There has been a long felt need for environmentally friendly compositions and methods for controlling or eradicating insect pests of agricultural significance, i.e., methods that are selective, environmentally inert, non-persistent, and biodegradable, and that fit well into insect pest management schemes.

The use of entomopathogenic fungi is one approach to meet these needs. The efficacy of entomopathogenic fungi such as *Metarhizium* spp. towards a variety of insect species is known (See International Patent Publication Number WO 2017/066094). However, the practical use of entomopathogenic fungi in agriculture depends on delivery of a viable propagule to the field, and the ability of that propagule to survive in the environment for a sufficient length of time to intercept the targeted insect pests. Fungal microsclerotia (MS) are one such propagule, but current practices for processing MS from culture to a formulated product/ingredient include steps damaging to the integrity of the propagule. Current processing steps include blending (for size reduction needed e.g. for spray-drying) and grinding (to break up filtercakes of MS or MS plus additives such as clay particles). Such treatment causes instability if MS thus processed are exposed to humidity, requiring storage under dry conditions for preservation of activity. This severely limits the utility of the MS in applications such as a seed treatment, where stability for months if not years is desirable. The degree of damage during processing is related to a lack of establishment in soil, a prerequisite for efficacy of an in-furrow or seed treatment product. These drawbacks to processing MS by conventional means have not been anticipated or documented in the patent or scientific literature. Thus, there is a need for compositions and methods of stably encapsulating entomopathogenic fungal propagules.

### SUMMARY OF THE INVENTION

The invention provides a method of preparing fungal entomopathogenic microsclerotia composition from a culture of microsclerotia comprising:
a) mixing the microsclerotia with an alginate salt solution to form a mixture of the microsclerotia and the alginate salt solution; and
b) adding the mixture to a multivalent cation solution to form an alginate bead encapsulating the microsclerotia;

wherein the microsclerotia in the alginate bead have enhanced stability;
and wherein the fungal entomopathogenic microsclerotia comprises at least one strain selected from the group consisting of:
   (i) the fungal entomopathogen deposited as Patent Deposit No. NRRL 67073;
   (ii) the fungal entomopathogen deposited as Patent Deposit No. NRRL 67074; and
   (iii) the fungal entomopathogen deposited as Patent Deposit No. NRRL 67075.

The invention further provides a composition comprising at least one entomopathogenic fungal strain encapsulated in an alginate bead; wherein the entomopathogenic fungal strain is selected from the group consisting of:
(i) the fungal entomopathogen deposited as Patent Deposit No. NRRL 67073;
(ii) the fungal entomopathogen deposited as Patent Deposit No. NRRL 67074; and
(iii) the fungal entomopathogen deposited as Patent Deposit No. NRRL 67075.

Yet further provided by the invention is a composition comprising at least one microsclerotium of at least one entomopathological fungal strain encapsulated in an alginate bead; wherein the entomopathogenic fungal strain is selected from the group consisting of:
(i) the fungal entomopathogen deposited as Patent Deposit No. NRRL 67073;
(ii) the fungal entomopathogen deposited as Patent Deposit No. NRRL 67074; and
(iii) the fungal entomopathogen deposited as Patent Deposit No. NRRL 67075.

Further aspects and preferred embodiments are defined in the dependent claims. Any aspects, embodiments and examples of the present disclosure which do not fall under the scope of the appended claims do not form part of the invention and are merely provided for illustrative purposes.

### SUMMARY OF TECHNICAL TEACHINGS

The invention is defined in the appended claims. Any of the following aspects not falling within the scope of said claims do not form part of the invention.

One aspect of the disclosure relates to a composition comprising an entomopathogenic fungal strain selected from *Metarhizium robertsii and Metarhizium anisopliae.* In certain aspects of the disclosure, the fungal entomopathogen comprises a spore, a microsclerotia, hyphae, a mycelium, or a conidia. In some aspects of the disclosure, a fungal entomopathogen has insecticidal activity.

In one aspect, the disclosure relates to a composition for increasing resistance to a plant pest, pathogen, or insect or for increasing plant health and/or yield comprising one or more entomopathogenic fungal strains selected from the group consisting of *Metarhizium robertsii* 15013-1 (NRRL 67073), *Metarhizium robertsii* 23013-3 (NRRL 67075), *Metarhizium anisopliae* 3213-1 (NRRL 67074), or any combinations thereof. In another aspect, the disclosure relates to a composition comprising an agriculturally accepted carrier and a fungal entomopathogen selected from the group consisting *of Metarhizium robertsii* 15013-1, *Metarhizium robertsii* 23013-3, *Metarhizium anisopliae* 3213-1, or any combinations thereof. In a further aspect of the disclosure, the fungal entomopathogen comprises a spore, conidia, or microsclerotia. In another aspect, the disclosure relates to a composition comprising one or more entomopathogenic fungal strains selected from the group consisting of *Metarhizium robertsii* 15013-1 (NRRL 67073), *Metarhizium robertsii* 23013-3 (NRRL 67075), *Metarhizium anisopliae* 3213-1 (NRRL 67074), mutants of these strains, a metabolite or combination of metabolites produced by a strain disclosed herein that exhibits insecticidal activity towards a plant pest, pathogen or insect, or any combinations thereof.

In yet another aspect, the disclosure relates to a composition comprising at least two entomopathogenic fungal strains selected from the group consisting *of Metarhizium robertsii* 15013-1, *Metarhizium robertsii* 23013-3, *Metarhizium anisopliae* 3213-1, or any combination thereof, in an effective amount to achieve an effect of inhibited growth of a plant pathogen, pest or insect. In another aspect of the disclosure, the composition comprises a fungal entomopathogen and an alginate salt. In further aspects of the disclosure, the composition comprising a fungal entomopathogen and an alginate salt may be formed into a bead. In some aspects of the disclosure, the beads may be formed by immersion of a mixture of fungal propagule and sodium alginate or chemically modified alginate into a solution containing a multivalent cation. In certain aspects of the disclosure, the multivalent cation may be provided as a calcium salt. In some aspects of the disclosure, the multivalent cation solution comprises a salt of calcium, wherein the salt of calcium further comprises carbonate, chloride, lactate, or gluconate. In certain aspects of the disclosure, the formed beads may range in diameter from 0.05mm to 10mm. In another aspect of the disclosure, the beads may be dried. In some aspects of the disclosure, the beads are greater than 1mm in diameter (a "macrobead"). In another aspect of the disclosure, the beads are equal to or less than 1mm (a "microbead"). In certain aspects of the disclosure, the dried beads may have a reduced size after drying. In other aspects of the disclosure, the dried beads may be broken into smaller fragments, which then may be applied to a plant or plant part. In some aspects of the disclosure, the beads are applied to a seed, plant, plant part, or applied to the soil near a seed, plant, or plant part.

In another aspect of the disclosure, the composition comprises a fungal entomopathogen and carrageenan. In further aspects of the disclosure, the composition comprising a fungal entomopathogen and carrageenan may be formed into a bead. In some aspects of the disclosure, the beads may be formed by immersion of a mixture of fungal propagule and carrageenan into a solution containing a divalent cation. In certain aspects of the disclosure, the divalent cation may be Ca²⁺. In some aspects of the disclosure, the multivalent cation solution comprises a salt of calcium, wherein the salt of calcium further comprises carbonate, chloride, lactate, or gluconate. In some aspects of the disclosure, the beads may be formed by immersion of a mixture of fungal propagule and carrageenan into a solution containing a monovalent cation. In certain aspects of the disclosure, the monovalent cation may be K²⁺. In certain aspects of the disclosure, the formed beads may range in diameter from 0.05mm to 10mm. In another aspect of the disclosure, the beads may be dried. In certain aspects of the disclosure, the dried beads may have a reduced size after drying.

Certain aspects of the disclosure relate to a process for preparing fungal entomopathogenic microsclerotia from a culture of microsclerotia comprising mixing the microsclerotia with an alginate salt solution to form a mixture of the microsclerotia and the alginate salt solution; and adding the mixture to a multivalent cation solution to form an alginate bead encapsulating the microsclerotia wherein the microsclerotia in the alginate bead have enhanced stability. In a further aspect of the disclosure, the method includes collecting the microsclerotia from the culture prior to mixing with the alginate salt solution, wherein the collecting may be performed by filtration, centrifugation, or by other means. In some aspects of the disclosure, the alginate beads encapsulating the microsclerotia have increased stability, increased germination, or increased establishment in the soil compared to microsclerotia not encapsulated in an alginate bead.

In another aspect of the disclosure, the compositions and methods relate to alginate beads encapsulating the microsclerotia that may be applied to the soil proximate to a plant or plant part prior to, simultaneous to, or after planting the plant or plant part. In another aspect of the disclosure, the alginate beads encapsulating the microsclerotia may be applied directly to a plant or plant part, wherein the alginate beads encapsulating the microsclerotia increase resistance to a plant pathogen, pest, or insect.

In another aspect of the disclosure, a composition disclosed herein further comprises a biocontrol agent or plant growth promoting agent selected from the group consisting of bacteria, fungi, yeast, protozoans, viruses, entomopathogenic nematodes, botanical extracts, proteins, secondary metabolites, and inoculants.

In another aspect of the disclosure, a composition comprises a fungal entomopathogen, an alginate salt, and one or more agrochemically active compounds selected from the group consisting of: an insecticide, a fungicide, a bactericide, and a nematicide. In one aspect of the disclosure, the fungicide comprises a fungicide composition selected from the group consisting of azoxystrobin, thiabendazole, fludioxonil, metalaxyl, tebuconazole, prothioconazole, ipconazole, penflufen, and sedaxane. In another aspect of the disclosure, a composition comprises a fungal entomopathogen, wherein the fungal entomopathogen is resistant to a fungicide. In another aspect of the disclosure, a composition comprises a fungal entomopathogen, wherein the fungal entomopathogen retains insecticidal activity in the presence of a fungicide. In still another aspect of the disclosure, the composition further comprises a compound selected from the group consisting of a safener, a lipochitooligosaccharide, an isoflavone, a benzoxazinoid, and a ryanodine receptor modulator.

In another aspect of the disclosure, a composition disclosed herein further comprises at least one seed, plant or plant part. In one aspect of the disclosure, the seed, plant, or plant part is genetically modified or a transgenic seed, plant or plant part. In a further aspect of the disclosure, the genetically modified or transgenic seed, plant, or plant part comprises an insecticidal trait derived from a plant, bacteria, non-*Bt* bacteria, an archea, an insect, or animal. In some aspects of the disclosure, the insecticidal trait comprises a Coleopteran insecticidal trait. In some aspects of the disclosure, an insecticidal trait may include a *Bt* trait, a non-*Bt* trait, and/or an RNAi trait. In some aspects, the compositions disclosed herein are applied as a seed coating, an in-furrow application, or as a foliar application.

In one aspect, a composition disclosed controls one or more plant pathogens, pests, or insects or inhibits the growth of one or more plant pathogens, pest or insects including, but not limited to, bacteria, a fungus, a virus, protozoa, a nematode or an arthropod. In one aspect, a composition disclosed herein controls or inhibits the growth of an insect, including, but not limited to a Coleopteran, Hemipteran, or Lepidopteran insect. In still another aspect, a composition disclosed herein controls or inhibits the growth of *Diabrotica* spp., including *Diabrotica virgifera virgifera.*

In another aspect, a composition disclosed herein is an effective amount to provide pesticidal activity to bacteria, plants, plant cells, tissues and seeds. In another aspect of the disclosure, the composition is an effective amount to provide pesticidal activity to Coleopteran or Lepidopteran insects. In still another aspect of the disclosure, the composition is an effective amount to provide pesticidal activity to *Diabrotica* spp., including *Diabrotica virgifera virgifera.*

In another aspect, a composition disclosed herein is effective to improve stability and viability of isolated fungal propagules.

In another aspect, the disclosure relates to a method comprising applying a composition comprising one or more entomopathogenic fungal strains selected from the group consisting *of Metarhizium robertsii* 15013-1, *Metarhizium robertsii* 23013-3, *Metarhizium anisopliae* 3213-1, or any combination thereof.

In another aspect, the disclosure relates to a method comprising applying a composition comprising one or more entomopathogenic fungal strains selected from the group consisting *of Metarhizium robertsii* 15013-1, *Metarhizium robertsii* 23013-3, *Metarhizium anisopliae* 3213-1, or any combination thereof to a seed, a plant, plant part or soil in an effective amount to achieve an effect selected from the group consisting of: inhibit a plant pathogen, pest, or insect or to prevent damage to a plant by a pathogen, pest, or insect, improve plant performance, improve plant yield, improve plant vigor, increase phosphate availability, increase production of a plant hormone, increase root formation, increase shoot height in a plant, increase leaf length of a plant, increase flower bud formation of a plant, increase total plant fresh weight, increase total plant dry weight, and increase seed germination.

In yet another aspect, the disclosure relates to a method comprising applying a composition comprising at least two entomopathogenic fungal strains selected from the group consisting of *Metarhizium robertsii* 15013-1, *Metarhizium robertsii* 23013-3, *Metarhizium anisopliae* 3213-1, or any combination thereof to a seed, a plant, plant part or soil in an effective amount to achieve an effect selected from the group consisting of: to inhibit a plant pathogen, pest, or insect, to prevent damage to a plant by a pathogen, pest, or insect, improve plant performance, improve plant yield, improve plant vigor, increase phosphate availability, increase production of a plant hormone, increase root formation, increase shoot height in a plant, increase leaf length of a plant, increase flower bud formation of a plant, increase total plant fresh weight, increase total plant dry weight, and increase seed germination.

In another aspect, the methods disclosed herein further comprise applying a composition further comprising a biocontrol agent, wherein the biocontrol agent selected from the group consisting of bacteria, fungi, yeast, protozoans, viruses, entomopathogenic nematodes, botanical extracts, proteins, secondary metabolites, and inoculants.

In yet another aspect, the methods disclosed herein further comprise applying a composition further comprising at least two strains are selected from the group consisting of: *Metarhizium robertsii* 15013-1, *Metarhizium robertsii* 23013-3, *Metarhizium anisopliae* 3213-1 and combinations thereof.

In yet another aspect, the methods disclosed herein further comprise applying a composition comprising one or more agrochemically active compounds selected from the group consisting of: an insecticide, a fungicide, a bactericide, and a nematicide. In one aspect of the disclosure, the fungicide comprises a fungicide composition selected from the group consisting of azoxystrobin, thiabendazole, fludioxonil, metalaxyl, tebuconazole, prothioconazole, ipconazole, penflufen, sedaxane. In another aspect of the disclosure, a composition comprises a fungal entompathogen, wherein the fungal entomopathogen is resistant to a fungicide. In another aspect of the disclosure, a composition comprises a fungal entompathogen, wherein the fungal entomopathogen retains insecticidal activity in the presence of a fungicide.

In still another aspect, the methods disclosed herein further comprise applying a composition further comprising a compound selected from the group consisting of a safener, a lipochitooligosaccharide, a benzoxazinoid, an isoflavone, and a ryanodine receptor modulator.

In another aspect, the methods disclosed herein further comprise applying the composition in an effective amount to inhibit growth of a plant pathogen, including but not limited to bacteria, a fungus, a nematode, an insect, a virus, and a protozoan.

In another aspect, the methods disclosed herein further comprise applying the composition in an effective amount to provide pesticidal activity to bacteria, plants, plant cells, tissues and seeds. In another aspect of the disclosure, the composition is an effective amount to provide pesticidal activity to Coleopteran, Hemipteran or Lepidopteran insects. In still another aspect of the disclosure, the composition is an effective amount to provide pesticidal activity to *Diabrotica* spp., including *Diabrotica virgifera virgifera.*

In another aspect, the methods disclosed herein relate to increasing durability of a *Coleopteran* insecticidal trait of a genetically modified or transgenic seed, plant part, or plant to a plant pathogen, a pest, or an insect comprising inoculating a genetically modified or transgenic seed, plant part, or plant with a composition comprising a fungal entomopathogen selected from the group consisting of *Metarhizium robertsii* 15013-1, *Metarhizium robertsii* 23013-3, and *Metarhizium anisopliae* 3213-1, wherein the genetically modified or transgenic seed, plant part or plant comprises a *Coleopteran* insecticidal trait.

### DESCRIPTION OF THE DRAWINGS

**FIG. 1****.** Shows plasmid pJS993 for production of Metarhizium reporter strain expressing ZsGreen.
**FIG. 2****.** Shows the effect of storage on stability and viability of conventionally-produced microsclerotia. Microsclerotia retained by a 100# filter (> 150µm in size) or which passed through a 200# filter (<75µm in size) were tested.
**FIG. 3****.** Shows microsclerotia (150µm fraction) encapsulated into an alginate bead. At top, brightfield, at bottom fluorescence microscopic images. Scale bar 500µm.
**FIG. 4****.** Shows microsclerotia germinating from within a dried alginate bead, after 24h in contact with water agar (fluorescence microscopy, black-and-white image).
**FIG. 5****.** Shows metarhizium colonies growing on potato dextrose agar (PDA), from four undried beads (left) and four dried beads (right).
**FIG. 6****.** Shows mean conidiospore production of *Metarhizium* Met26 microsclerotia held over time at 20°C under low (o) or high (•) relative humidity. Beads contained 8.3% (w/v) microsclerotia prior to drying. Error bars are SEM.
**FIG. 7****.** Shows mean conidiospore production potential (CPP) of dried alginate beads held over time at 20°C under low (∘) or high (•) relative humidity. Beads contained 8.3% (w/v) microsclerotia prior to drying. Error bars are SEM.
**FIG. 8****.** Shows hyphal emergence from a dried alginate bead after 48h in water agar, following 7d at high humidity, 20°C (1:1 alginate/microsclerotia, >150µm fraction). Fluorescence microscopy, scale bar 500µm.
**FIG. 9****.** Shows colony-forming units (CFU) of *Metarhizium* present in a topsoil-sand mixture at one or four weeks following inoculation of a single dried alginate bead. Prior to drying, beads contained either 3% or 8.3% (w/v) Met26 microsclerotia, or 8% (w/v) untransformed 15013-1 microsclerotia.
**FIG. 10****.** Shows mean conidiospore production potential (CPP) of dried alginate microbeads held over time at 25°C and 55% humidity. Open symbols (∘) indicate encapsulated microsclerotia, closed symbols (•) unencapsulated. CPP of unencapsulated material was not measured after eight weeks. Beads contained 4.0% (w/v) microsclerotia prior to drying.

### DETAILED DESCRIPTION

The invention is defined in the appended claims. Any aspects, embodiments and examples of the present disclosure which do not fall under the scope of the appended claims do not form part of the invention and are merely provided for illustrative purposes. Numeric ranges are inclusive of the numbers defining the range.

The headings provided are not limitations of the various aspects or embodiments, which can be had by reference to the specification.

Other definitions of terms may appear throughout the specification.

The article "a" and "an" are used to refer to one or more than one (i.e., to at least one) of the grammatical object of the article. For example, "an element" means one or more element.

As used herein, "administer" refers to the action of introducing a strain, an alginate bead encapsulating fungal entomopathogenic microsclerotia, and/or a composition containing such to an environment for pathogen, pest, or insect inhibition or to improve plant performance.

As used herein, "alginate salt" refers to any salt of alginic acid, an anionic polysaccharide material. Alginates are polyanionic linear block copolymers containing blocks of (1,4)-linked β-d-mannuronic and α-l-guluronic acids. Alginate is commonly extracted from seaweed, and is also produced by two kinds of bacteria, *Pseudomonas* and *Azotobacter.* Alginates may vary in composition, structure and properties (e.g. viscosity) depending on their source, and beads formed from different alginates may have different properties (e.g. mechanical integrity, porosity or permeability).

As used herein, the term "chemically modified" refers to the chemical derivatization of a material. In one aspect of the disclosure, chemically modified includes the esterification of carboxyl groups of alginate with propylene glycol to yield propylene glycol alginates.

As used herein, the term "agrochemically active compounds" refers to any substance that is or may be customarily used for treating plants including, but not limited to, fungicides, bactericides, insecticides, acaricides, nematicides, molluscicides, safeners, plant growth regulators, and plant nutrients, as well as, microorganisms. Compositions disclosed herein may comprise fungicides which may include, but are not limited to, the respiration inhibitors, such as azoxystrobin, which target complex III of mitochondrial electron transport; tubulin inhibitors, such as thiabendazole, which bind to beta-tubulin; the osmotic stress related-kinase inhibitor fludioxonil; an RNA polymerase inhibitor of Oomycetes, a group of fungal-like organisms, such as metalaxyl; inhibitors of sterol biosynthesis, which include inhibitors of the C-14 demethylase of the sterol biosynthesis pathway (commonly referred to as demethylase inhibitors or DMIs), such as tebuconazole, prothioconazole, and ipconazole; a respiration inhibitor which targets complex II mitochondrial electron transport, such as a penflufen; a respiration inhibitor which targets complex II mitochondrial electron transport, such as sedaxane. Other classes of fungicides with different or similar modes of action can be found at frac.info/docs/default-source/publications/frac-code-list/frac-code-list-2016.pdf?sfvrsn=2 (which can be accessed on the world-wide web using the "www" prefix; See Hirooka and Ishii (2013), Journal of General Plant Pathology)*.* A fungicide may comprise all or any combination of different classes of fungicides as described herein. In certain aspects, a composition disclosed herein comprises azoxystrobin, thiabendazole, fludioxonil, and metalaxyl. In another aspect, a composition disclosed herein comprises a tebuconazole. In another aspect, a composition disclosed herein comprises prothioconazole, metalaxyl, and penflufen. In another aspect, a composition disclosed herein comprises ipconazole and metalaxyl. In another aspect, a composition disclosed herein comprises sedaxane. As used herein, a composition may be a liquid, a heterogeneous mixture, a homogeneous mixture, a powder, a solution, a dispersion or any combination thereof.

As used herein, "effective amount" refers to a quantity of an entomopathogenic fungal strain, an alginate bead encapsulating fungal entomopathogenic microsclerotia, and/or a composition containing such sufficient to inhibit growth of a pathogenic microorganism or to impede the rate of growth of the pathogenic microorganism. In another aspect of the disclosure, the term "effective amount" refers to a quantity of an entomopathogenic fungal strain, an alginate bead encapsulating fungal entomopathogenic microsclerotia, and/or a composition containing such sufficient to improve plant performance. In another aspect of the disclosure, the term "effective amount" refers to a quantity of an entomopathogenic fungal strain, an alginate bead encapsulating fungal entomopathogenic microsclerotia, and/or a composition containing such sufficient to control, kill, inhibit, and reduce the number, emergence, or growth of a pathogen, pest, or insect. In another aspect of the disclosure, the term "effective amount" refers to a quantity of an entomopathogenic fungal strain, an alginate bead encapsulating fungal entomopathogenic microsclerotia, and/or a composition containing such sufficient to prevent damage from a pathogen, pest, or insect. One skilled in the art will recognize that an effective amount of an entomopathogenic fungal strain, an alginate bead encapsulating fungal entomopathogenic microsclerotia, and/or a composition containing such may not reduce the numbers of pathogens, pests or insects, but is effective in decreasing damage to plants and/or plant parts as a result of a pathogen, pest or insect. For example, a pesticidally effective amount may reduce pathogen, pest or insect emergence, or damage to seeds, roots, shoots, or foliage of plants that are treated compared to those that are untreated.

As used herein, the term "enhanced stability" refers to preservation of viability under ambient conditions compared to a non-encapsulated microsclerotia.

As used herein, the term "entomopathogenic fungal strain" or "entomopathogenic fungal composition" includes, but is not limited to conidia spores, spores, mycelia, microsclerotia, and/or any other life cycle stage of a fungal entomopathogen.

As used herein, the term "fungal propagule" refers to and includes fungal microsclerotia.

As used herein, the term "environment of a plant or plant part" is intended to mean the area surrounding the plant or plant part, including but not limited to the soil, the air, or in-furrow. The environment of a plant or plant part may be in close proximity, touching, adjacent to, or in the same field as the plant or plant part. The compositions described herein may be applied to the environment of the plant or plant part as a seed treatment, as a foliar application, as a granular application, as a soil application, or as an encapsulated application. As used herein, "in-furrow" is intended to mean within or near the area where a seed is planted. The compositions disclosed herein may be applied in-furrow concurrently or simultaneously with a seed. In another aspect, the compositions disclosed herein may be applied sequentially, either before or after a seed is planted.

As used herein, the term "pathogen, pest, or insect" includes but is not limited to pathogenic fungi, bacteria, mites, ticks, pathogenic microorganisms, and nematodes, as well as insect from the orders *Coleoptera, Lepidoptera, Mallophaga, Homoptera, Hemiptera, Orthroptera, Thysanoptera, Dermaptera, Isoptera, Anoplura, Siphonatpera, Trichoptera, Diptera,* and others, including but not limited to *Diabrotica virgifera virgifera, Diabrotica undecimpunctata howardi, Diabrotica speciosa,* and *Diabrotica barberi.*

Aspects disclosed herein are useful in the inhibition of larvae and adults of the order Coleoptera from the families Anthribidae, Bruchidae and Curculionidae (including, but not limited to: *Anthonomus grandis* Boheman (boll weevil); *Lissorhoptrus oryzophilus* Kuschel (rice water weevil); *Sitophilus granarius* Linnaeus (granary weevil); *S. oryzae* Linnaeus (rice weevil); *Hypera punctata* Fabricius (clover leaf weevil); *Cylindrocopturus adspersus* LeConte (sunflower stem weevil); *Smicronyx fulvus* LeConte (red sunflower seed weevil); S. *sordidus* LeConte (gray sunflower seed weevil); *Sphenophorus maidis* Chittenden (maize billbug)); flea beetles, cucumber beetles, rootworms, leaf beetles, potato beetles and leafminers in the family Chrysomelidae (including, but not limited to: *Leptinotarsa decemlineata* Say (Colorado potato beetle); *Diabrotica virgifera virgifera* LeConte (western corn rootworm); *D. barberi* Smith and Lawrence (northern corn rootworm); *D. undecimpunctata howardi* Barber (southern corn rootworm); *Chaetocnema pulicaria* Melsheimer (corn flea beetle); *Phyllotreta cruciferae* Goeze (Crucifer flea beetle); *Phyllotreta striolata* (stripped flea beetle); *Colaspis brunnea* Fabricius (grape colaspis); *Oulema melanopus* Linnaeus (cereal leaf beetle); *Zygogramma exclamationis* Fabricius (sunflower beetle)); beetles from the family Coccinellidae (including, but not limited to: *Epilachna varivestis* Mulsant (Mexican bean beetle)); chafers and other beetles from the family Scarabaeidae (including, but not limited to: *Popillia japonica* Newman (Japanese beetle); *Cyclocephala borealis* Arrow (northern masked chafer, white grub); *C. immaculata* Olivier (southern masked chafer, white grub); *Rhizotrogus majalis* Razoumowsky (European chafer); *Phyllophaga crinita* Burmeister (white grub); *Ligyrus gibbosus* De Geer (carrot beetle)); carpet beetles from the family Dermestidae; wireworms from the family Elateridae, *Eleodes* spp., *Melanotus* spp.; *Conoderus* spp.; *Limonius* spp.; *Agriotes* spp.; *Ctenicera* spp.; *Aeolus* spp.; fruit flies of the family Tephritidae (including, but not limited to, the olive fruit fly Bactrocera oleae), bark beetles from the family Scolytidae and beetles from the family Tenebrionidae.

Methods for measuring pesticidal activity are well known in the art. See, for example, Czapla and Lang, (1990) J. Econ. Entomol. 83:2480-2485; Andrews, et al., (1988) Biochem. J. 252:199-206; Marrone, et al., (1985) J. of Economic Entomology 78:290-293 and US Patent Number 5,743,477. Generally, the pesticide is mixed and used in feeding assays. See, for example Marrone, et al., (1985) J. of Economic Entomology 78:290-293. Such assays can include contacting plants with one or more pests and determining the plant's ability to survive and/or cause the death of the pests.

As used herein, the term "plant" refers to all plants, plant parts, and plant populations, such as desirable and undesirable wild plants, cultivars, transgenic plants, and plant varieties (whether or not protectable by plant variety or plant breeder's rights). Cultivars and plant varieties can be plants obtained by conventional propagation and breeding methods that can be assisted or supplemented by one or more biotechnological methods such as by use of double haploids, protoplast fusion, random and directed mutagenesis, molecular or genetic markers or by bioengineering and genetic engineering methods.

The aspects disclosed herein may generally be used for any plant species, including, but not limited to, monocots and dicots. Examples of plants of interest include, but are not limited to, corn (*Zea mays*)*, Brassica* sp. (e.g., *B. napus, B. rapa, B. juncea),* particularly those *Brassica* species useful as sources of seed oil, alfalfa (*Medicago sativa*)*,* rice (*Oryza sativa*)*,* rye (*Secale cereale*)*,* sorghum (*Sorghum bicolor, Sorghum vulgare*)*,* millet (e.g., pearl millet (*Pennisetum glaucum*), proso millet (*Panicum miliaceum*)*,* foxtail millet (*Setaria italica*), finger millet (*Eleusin*e *coracana*)), sunflower (*Helianthus annuus*)*,* safflower (*Carthamus tinctorius*)*,* wheat (*Triticum aestivum*)*,* soybean (*Glycine max*)*,* tobacco (*Nicotiana tabacum*)*,* potato (*Solanum tuberosum*)*,* peanuts (*Arachis hypogaea*), cotton (*Gossypium barbadense, Gossypium hirsutum),* sweet potato (*Ipomoea batatus*)*,* cassava (*Manihot esculenta*), coffee (*Coffea* spp.), coconut (*Cocos nucifera*), pineapple (*Ananas comosus),* citrus trees (*Citrus* spp.), cocoa (*Theobroma cacao),* tea (*Camellia sinensis),* banana (*Musa* spp.), avocado (*Persea americana),* fig (*Ficus casica*), guava (*Psidium guajava*), mango (*Mangifera indica),* olive (*Olea europaea),* papaya (*Carica papaya*), cashew (*Anacardium occidentale),* macadamia (*Macadamia integrifolia*), almond (*Prunus amygdalus*), sugar beets (*Beta vulgaris),* sugarcane (*Saccharum* spp.), oats, barley, vegetables, ornamentals, and conifers.

As used herein, the term "plant parts" refers to all above ground and below ground parts and organs of plants such as shoot, leaf, blossom and root, whereby for example leaves, needles, stems, branches, blossoms, fruiting bodies, fruits and seeds, as well as roots, tubers, corms and rhizomes are included. Crops and vegetative and generative propagating material, for example, cuttings, corms, rhizomes, tubers, runners and seeds are also plant parts.

As used herein, the term "spore" includes, but is not limited to conidia spores, blastospores, mycelia, microsclerotia, and/or any other life cycle stage of a fungal entomopathogen. An "aerial conidispore" (AC) refers to conidiaspores formed by the asexual developmental cycle on the surface of an agar medium, or other solid substrate of appropriate composition. As used herein, term "submerged spores" refers to submerged conidiaspores and/or blastospores that develop in liquid culture.

As used herein, the term "wet microsclerotia" as used herein refers to a suspension of microsclerotia in an aqueous solution prior to encapsulation, for example. In some aspects of the disclosure, wet microsclerotia are previously subjected to drying then re-suspended in an aqueous solution. In some aspects of the disclosure, wet microsclerotia are not subjected to any drying. In some aspects of the disclosure, wet microsclerotia are a direct product of a liquid fermentation process. In one aspect of the disclosure, a wet microsclerotia preparation is encapsulated by alginate beads. In another aspect of the disclosure, a dry microsclerotia preparation is encapsulated by alginate beads.

As used herein, the term "viable" refers to a microbial cell, propagule, or spore that is metabolically active or able to differentiate. Thus, propagules, such as microsclerotia or spores, are "viable" when they are dormant and capable of germinating.

Biological control of insect pests of agricultural significance using a microbial agent, such as fungi, bacteria, or another species of insect affords an environmentally friendly and commercially attractive alternative to synthetic chemical pesticides. Generally speaking, the use of biopesticides presents a lower risk of pollution and environmental hazards and biopesticides provide greater target specificity than is characteristic of traditional broad-spectrum chemical insecticides. In addition, biopesticides often cost less to produce and thus improve economic yield for a wide variety of crops.

Certain species of microorganisms of the genus *Bacillus* are known to possess pesticidal activity against a range of insect pests including *Lepidoptera, Diptera, Coleoptera, Hemiptera* and others. *Bacillus thuringiensis (Bt)* and *Bacillus popilliae* are among the most successful biocontrol agents discovered to date. Insect pathogenicity has also been attributed to strains of *B. larvae, B. lentimorbus, B. sphaericus* and *B. cereus.* Microbial insecticides, particularly those obtained from *Bacillus* strains, have played an important role in agriculture as alternatives to chemical pest control.

Crop plants have been developed with enhanced insect resistance by genetically engineering crop plants to produce pesticidal proteins from *Bacillus.* For example, corn and cotton plants have been genetically engineered to produce pesticidal proteins isolated and/or engineered from strains of *Bt* (herein referred to as a *"Bt* trait"). These genetically modified crops are now widely used in agriculture and have provided the farmer with an environmentally friendly alternative to traditional insect-control methods. While they have proven to be very successful commercially, these genetically modified, insect-resistant crop plants provide resistance to only a narrow range of the economically important insect pests. In some cases, insects can develop resistance to different insecticidal compounds, which raises the need to identify alternative biological control agents for pest control.

The aspects disclosed herein relate to entomopathogenic fungal strains, entomopathogenic fungal compositions, methods of processing and encapsulating fungal propagules, methods of using the propagules, methods of making and using an alginate bead encapsulating fungal entomopathogenic microsclerotia, and/or a composition containing such. In one aspect of the disclosure, the entomopathogenic strains and alginate beads encapsulating fungal entomopathogenic microsclerotia have insecticidal activity and may find use in inhibiting, controlling, or killing a pathogen, pest, or insect, including, but is not limited to, fungi, pathogenic fungi, bacteria, mites, ticks, pathogenic microorganisms, and nematodes, as well as insects from the orders *Coleoptera, Diptera, Hymenoptera, Lepidoptera, Mallophaga, Homoptera, Hemiptera Orthroptera, Thysanoptera, Dermaptera, Isoptera, Anoplura, Siphonaptera, Trichoptera, etc., particularly Coleoptera,* including but not limited to *Diabrotica virgifera virgifera, Diabrotica undecimpunctata howardi,* and *Diabrotica barberi,* and for producing compositions with pesticidal activity. In one aspect of the disclosure, the entomopathogenic fungal strain(s) encapsulated in an alginate bead are selected from the group consisting of: *Metarhizium robertsii* 15013-1, *Metarhizium robertsii* 23013-3, *Metarhizium anisopliae* 3213-1, and combinations thereof.

*Metarhizium robertsii* 15013-1 (NRRL 67073) was deposited on June 18, 2015 at the Agricultural Research Service Culture Collection (NRRL), 1815 North University Street, Peoria, Ill., 61604 and given accession number NRRL 67073. The deposits were made under the provisions of the Budapest Treaty on the International Recognition of the Deposit of Microorganisms for the Purposes of Patent Procedure.

*Metarhizium robertsii* 23013-3 (NRRL 67075) was deposited on June 18, 2015 at the Agricultural Research Service Culture Collection (NRRL), 1815 North University Street, Peoria, Ill., 61604 and given accession number NRRL 67075. The deposits were made under the provisions of the Budapest Treaty on the International Recognition of the Deposit of Microorganisms for the Purposes of Patent Procedure.

*Metarhizium anisopliae* 3213-1 (NRRL 67074) was deposited on June 18, 2015 at the Agricultural Research Service Culture Collection (NRRL), 1815 North University Street, Peoria, Ill., 61604 and given accession number NRRL 67074. The deposits were made under the provisions of the Budapest Treaty on the International Recognition of the Deposit of Microorganisms for the Purposes of Patent Procedure.

The *Metarhizium robertsii* 15013-1 (NRRL 67073), *Metarhizium robertsii* 23013-3 (NRRL 67075), and *Metarhizium anisopliae* 3213-1 (NRRL 67074) deposits will be maintained under the terms of the Budapest Treaty on the International Recognition of the Deposit of Microorganisms for the Purposes of Patent Procedure. The strains deposited with the NRRL on or about June 18, 2015 were taken from the deposit maintained by Pioneer Hi-Bred International, Inc., 7250 NW 62^{nd} Avenue, Johnston, Iowa 50131-1000. Access to these deposits will be available during the pendency of the application to the Commissioner of Patents and Trademarks and persons determined by the Commissioner to be entitled thereto upon request. Upon allowance of any claims in the application, the Applicant will make available to the public, pursuant to 37 C.F.R. § 1.808, sample(s) of the deposits. The deposits will be maintained in the NRRL depository, which is a public depository, for a period of 30 years, or 5 years after the most recent request, or for the enforceable life of the patent, whichever is longer, and will be replaced if it becomes nonviable during that period. Additionally, Applicant has satisfied all the requirements of 37 C.F.R. §§1.801 - 1.809, including providing an indication of the viability of the sample upon deposit.

Fungal microsclerotia (MS) harvested non-destructively from culture may be encapsulated in alginate beads. The beads containing MS may be dried under mild conditions (e.g. room temperature airflow) to yield small granules. MS will germinate from within these granules (or the parent beads), as evidenced by emergent hyphal growth on nutrient or water agar plates. Germination from dried beads occurs after storage at high (55%) humidity for several weeks at 20°C. Under these conditions, size-reduced MS produced by conventional processing are not viable. By comparison, microsclerotia encapsulated in alginate beads are stable for extended periods. In some aspects of the disclosure, fungal microsclerotia encapsulated in alginate beads are viable for at least 1, 5, 10, 15, 20, 25, 30, 35, 40, 45, 50, 55, 60, 65, 60, 75, 80, 85, 90, 95, 100, 105, 110 weeks. In some aspects of the disclosure, the fungal microsclerotia encapsulated in alginate beads are viable for at least one year.

Alternative calcium salts e.g. calcium gluconate can be employed in this process. In principle other encapsulants in which MS are incorporated with minimal damage due to processing, and which may be dried to low water activity with minimal damage to MS integrity, may be employed. Similarly, additives may be incorporated to enhance efficacy or stability, or additional biologicals may be incorporated.

In certain aspects of the disclosure, recovering and formulating a fungal entomopathogen (*Metarhizium spp.)* propagule from a liquid culture comprises harvesting a fermentation broth. A fermenter may be rinsed with about 1x to 2x the volume of a fermentation broth, and the diluted broth pooled with the neat broth.

In a conventional process, diluted entomopathogenic fungal material in a fermentation broth may be treated with an admixture of diatomaceous earth (DE Admix) or clays. A treated fermentation broth may be filtered to remove medium. A filter cake thus produced may be washed with water to remove unwanted medium constituents and non-microsclerotial material. A filter cake thus produced may be processed immediately or stored in a cold room until processing. A wet filter cake may be broken up and dried by various means for about 48 h to 5 days. A dried filter cake may be ground to create a final entomopathogenic fungal dried powder product.

Diluted entomopathogenic fungal material in a fermentation broth may be subjected to blending to reduce sclerotia size to assist with subsequent process steps. A blended fermentation broth may be treated with an admixture of diatomaceous earth (DE Admix) or clays. A treated fermentation broth may be filtered to remove medium. A filter cake thus produced may be washed with water to remove unwanted medium constituents and non-microsclerotial material. A filter cake thus produced may be processed immediately or stored in a cold room until processing. A wet filter cake may be broken up and dried by various means for about 48 h to 5 days. A dried filter cake may be ground to create a final entomopathogenic fungal dried powder product. Alternatively, a wet filter cake thus produced may be dried by other means, including spray drying.

Diluted entomopathogenic fungal material in a fermentation broth may be treated with alginate admixture.

One aspect of the disclosure relates to a composition comprising or consisting of or consisting essentially of an entomopathogenic fungal strain selected from the group consisting of: *Metarhizium robertsii* 15013-1, *Metarhizium robertsii* 23013-3, and *Metarhizium anisopliae* 3213-1 encapsulated in an alginate bead. In another aspect of the disclosure, the composition comprises, consists of, or consists essentially of at least two or more entomopathogenic fungal strains selected from the group consisting of: *Metarhizium robertsii* 15013-1, *Metarhizium robertsii* 23013-3, *Metarhizium anisopliae* 3213-1 encapsulated in an alginate bead. In a further aspect of the disclosure, the composition comprises, consists of, or consists essentially of the entomopathogenic fungal strains selected from the group consisting of: *Metarhizium robertsii* 15013-1, *Metarhizium robertsii* 23013-3, and *Metarhizium anisopliae* 3213-1 encapsulated in an alginate bead. In an aspect of the disclosure, a composition is a biologically pure culture of *Metarhizium robertsii* 15013-1, *Metarhizium robertsii* 23013-3, and *Metarhizium anisopliae* 3213-1 and combinations thereof encapsulated in an alginate bead.

One aspect of the disclosure relates to a composition comprising the entomopathogenic fungal strains disclosed herein encapsulated in an alginate bead and one or more compounds or agents selected from the group consisting of: agrochemically active compounds, biocontrol agents, lipo-chitooligosaccharide compounds (LCOs), benzoxazinoids, isoflavones, quinazolines, insecticidal compounds, azolopyrimidinylamines, polymeric compounds, ionic compound, substituted thiophenes, substituted dithiines, fluopyramm, enaminocarbonyl compounds, strigolactone compound, and dithiino-tetracarboximide compounds and combinations thereof.

A further aspect of the disclosure relates to the use of a first composition comprising the entomopathogenic fungal strains disclosed herein encapsulated in an alginate bead and a second composition comprising one or more compounds or agents selected from the group consisting of: agrochemically active compounds, biocontrol agents, lipochitooligosaccharide compounds (LCOs), benzoxazinoids, isoflavones, quinazolines, insecticidal compound, azolopyrimidinylamine, polymeric compounds, ionic compound, substituted thiophenes, substituted dithiines, fluopyramm, enaminocarbonyl compounds, strigolactone compound, and dithiino-tetracarboximide compounds and combinations thereof.

In one aspect, the disclosure relates to a composition comprising one or more entomopathogenic fungal strains disclosed herein encapsulated in an alginate bead and one or more biocontrol agents. As used herein, the term "biocontrol agent" ("BCA") includes bacteria, fungi or yeasts, protozoans, viruses, entomopathogenic nematodes, and botanical extracts, or products produced by microorganisms including proteins or secondary metabolite, and inoculants that have one or both of the following characteristics: (1) inhibits or reduces plant infestation and/or growth of pathogens, pests, or insects, including but not limited to pathogenic fungi, bacteria, and nematodes, as well as arthropod pests such as insects, arachnids, chilopods, diplopods, or that inhibits plant infestation and/or growth of a combination of plant pathogens, pests, or insects; (2) improves plant performance; (3) improves plant yield; (4) improves plant vigor; and (5) improves plant health.

In one aspect, the disclosure relates to a composition comprising an entomopathogenic fungal strain disclosed herein encapsulated in an alginate bead and an agrochemically active compound. Agrochemically active compounds are substances that are or may be used for treating or applying to a seed, a plant, plant part, or the environment of the seed or plant or plant part including but not limited to fungicides, bactericides, insecticides, acaricides, nematicides, molluscicides, safeners, plant growth regulators, plant nutrients, chemical entities with a known mechanism of action, additional microorganisms, and biocontrol agents.

In one aspect, a composition disclosed herein comprises one or more agrochemically active compounds, wherein one compound is chlorantraniliprole (Rynaxypyr^{®}). In another aspect of the disclosure, the composition comprises one or more agrochemically active compounds, wherein one compound is cyantraniliprole (Cyazypyr^{®}). In another aspect of the disclosure, the composition comprises both chlorantraniliprole and cyantraniliprole.

In an aspect, a first and a second composition disclosed herein can be applied at the same time to a seed. In another aspect of the disclosure, a first composition can be applied to a seed followed by application of a second composition to the seed. In yet another aspect of the disclosure, a second composition can be applied to a seed followed by application of a first composition to the seed. In another aspect of the disclosure, a first composition can be applied to a seed followed by application of a second composition to a plant. In yet another aspect of the disclosure, a second composition can be applied to a seed followed by application of a first composition to a plant. In another aspect of the disclosure, a first composition can be applied to a seed followed by application of a second composition to a plant part. In yet another aspect of the disclosure, a second composition can be applied to a seed followed by application of a first composition to a plant part. In another aspect of the disclosure, a first composition can be applied to a seed followed by application of a second composition to the environment of the seed. In yet another aspect of the disclosure, a second composition can be applied to a seed followed by application of a first composition to the environment of the seed. In another aspect of the disclosure, a first composition can be applied to a seed followed by application of a second composition to the environment of a plant. In yet another aspect of the disclosure, a second composition can be applied to a seed followed by application of a first composition to the environment of a plant. In another aspect of the disclosure, a first composition can be applied to a seed followed by application of a second composition to the environment of a plant part. In yet another aspect of the disclosure, a second composition can be applied to a seed followed by application of a first composition to the environment of a plant part.

In an aspect, a first and a second composition disclosed herein can be applied at the same time to a plant. In another aspect of the disclosure, a first composition can be applied to a plant followed by application of a second composition to the plant. In yet another aspect of the disclosure, a second composition can be applied to a plant followed by application of a first composition to the plant. In another aspect of the disclosure, a first composition can be applied to a plant followed by application of a second composition to a seed. In yet another aspect of the disclosure, a second composition can be applied to a plant followed by application of a first composition to a seed. In another aspect of the disclosure, a first composition can be applied to a plant followed by application of a second composition to a plant part. In yet another aspect of the disclosure, a second composition can be applied to a plant followed by application of a first composition to a plant part. In another aspect of the disclosure, a first composition can be applied to a plant followed by application of a second composition to the environment of a seed. In yet another aspect of the disclosure, a second composition can be applied to a plant followed by application of a first composition to the environment of a seed. In another aspect of the disclosure, a first composition can be applied to a plant followed by application of a second composition to the environment of the plant. In yet another aspect of the disclosure, a second composition can be applied to a plant followed by application of a first composition to the environment of the plant. In another aspect of the disclosure, a first composition can be applied to a plant followed by application of a second composition to the environment of a plant part. In yet another aspect of the disclosure, a second composition can be applied to a plant followed by application of a first composition to the environment of a plant part.

In an aspect, a first and a second composition disclosed herein can be applied at the same time to a plant part. In another aspect of the disclosure, a first composition can be applied to a plant part followed by application of a second composition to the plant part. In yet another aspect of the disclosure, a second composition can be applied to a plant part followed by application of a first composition to the plant part. In another aspect of the disclosure, a first composition can be applied to a plant part followed by application of a second composition to a seed. In yet another aspect of the disclosure, a second composition can be applied to a plant part followed by application of a first composition to a seed. In another aspect of the disclosure, a first composition can be applied to a plant part followed by application of a second composition to a plant. In yet another aspect of the disclosure, a second composition can be applied to a plant part followed by application of a first composition to a plant. In another aspect of the disclosure, a first composition can be applied to a plant part followed by application of a second composition to the environment of a seed. In yet another aspect of the disclosure, a second composition can be applied to a plant part followed by application of a first composition to the environment of a seed. In another aspect of the disclosure, a first composition can be applied to a plant part followed by application of a second composition to the environment of a plant. In yet another aspect of the disclosure, a second composition can be applied to a plant part followed by application of a first composition to the environment of a plant. In another aspect of the disclosure, a first composition can be applied to a plant part followed by application of a second composition to the environment of the plant part. In yet another aspect of the disclosure, a second composition can be applied to a plant part followed by application of a first composition to the environment of the plant part.

In an aspect, a first and a second composition disclosed herein can be applied at the same time to the environment of a seed. In another aspect of the disclosure, a first composition can be applied to the environment of a seed followed by application of a second composition to the environment of the seed. In yet another aspect of the disclosure, a second composition can be applied to the environment of a seed followed by application of a first composition to the environment of the seed. In another aspect of the disclosure, a first composition can be applied to the environment of a seed followed by application of a second composition to a seed. In yet another aspect of the disclosure, a second composition can be applied to the environment of a seed followed by application of a first composition to a seed. In another aspect of the disclosure, a first composition can be applied to the environment of a seed followed by application of a second composition to a plant. In yet another aspect of the disclosure, a second composition can be applied to the environment of a seed followed by application of a first composition to a plant. In another aspect of the disclosure, a first composition can be applied to the environment of a seed followed by application of a second composition to a plant part. In yet another aspect of the disclosure, a second composition can be applied to the environment of a seed followed by application of a first composition to a plant part. In another aspect of the disclosure, a first composition can be applied to the environment of a seed followed by application of a second composition to the environment of a plant. In yet another aspect of the disclosure, a second composition can be applied to the environment of a seed followed by application of a first composition to the environment of a plant. In another aspect of the disclosure, a first composition can be applied to the environment of a seed followed by application of a second composition to the environment of a plant part. In yet another aspect of the disclosure, a second composition can be applied to the environment of a seed followed by application of a first composition to the environment of a plant part.

In an aspect, a first and a second composition disclosed herein can be applied at the same time to the environment of a plant. In another aspect of the disclosure, a first composition can be applied to the environment of a plant followed by application of a second composition to the environment of the plant. In yet another aspect of the disclosure, a second composition can be applied to the environment of a plant followed by application of a first composition to the environment of the plant. In another aspect of the disclosure, a first composition can be applied to the environment of a plant followed by application of a second composition to a seed. In yet another aspect of the disclosure, a second composition can be applied to the environment of a plant followed by application of a first composition to a seed. In another aspect of the disclosure, a first composition can be applied to the environment of a plant followed by application of a second composition to a plant. In yet another aspect of the disclosure, a second composition can be applied to the environment of a plant followed by application of a first composition to a plant. In another aspect of the disclosure, a first composition can be applied to the environment of a plant followed by application of a second composition to a plant part. In yet another aspect of the disclosure, a second composition can be applied to the environment of a plant followed by application of a first composition to a plant part. In another aspect of the disclosure, a first composition can be applied to the environment of a plant followed by application of a second composition to the environment of a seed. In yet another aspect of the disclosure, a second composition can be applied to the environment of a plant followed by application of a first composition to the environment of a seed. In another aspect of the disclosure, a first composition can be applied to the environment of a plant followed by application of a second composition to the environment of a plant part. In yet another aspect of the disclosure, a second composition can be applied to the environment of a plant followed by application of a first composition to the environment of a plant part.

In an aspect, a first and a second composition disclosed herein can be applied at the same time to the environment of a plant part. In another aspect of the disclosure, a first composition can be applied to the environment of a plant part followed by application of a second composition to the environment of the plant part. In yet another aspect of the disclosure, a second composition can be applied to the environment of a plant part followed by application of a first composition to the environment of the plant part. In another aspect of the disclosure, a first composition can be applied to the environment of a plant part followed by application of a second composition to a seed. In yet another aspect of the disclosure, a second composition can be applied to the environment of a plant part followed by application of a first composition to a seed. In another aspect of the disclosure, a first composition can be applied to the environment of a plant part followed by application of a second composition to a plant. In yet another aspect of the disclosure, a second composition can be applied to the environment of a plant part followed by application of a first composition to a plant. In another aspect of the disclosure, a first composition can be applied to the environment of a plant part followed by application of a second composition to the environment of a seed. In yet another aspect of the disclosure, a second composition can be applied to the environment of a plant part followed by application of a first composition the environment of a seed. In another aspect of the disclosure, a first composition can be applied to the environment of a plant part followed by application of a second composition to the environment of a plant. In yet another aspect of the disclosure, a second composition can be applied to the environment of a plant part followed by application of a first composition to the environment of a plant.

In one aspect, the disclosure relates to the use of the entomopathological fungal strains disclosed herein encapsulated in an alginate bead with a composition comprising an insecticidal protein from *Pseudomonas* sp. such as PSEEN3174 (Monalysin; (2011) PLoS Pathogens 7:1-13); from *Pseudomonas protegens* strain CHAO and Pf-5 (previously *fluorescens*) (Pechy-Tarr, (2008) Environmental Microbiology 10:2368-2386; GenBank Accession No. EU400157); from *Pseudomonas taiwanensis* (Liu, et al., (2010) J. Agric. Food Chem., 58:12343-12349) and from *Pseudomonas pseudoalcligenes* (Zhang, et al., (2009) Annals of Microbiology 59:45-50 and Li, et al., (2007) Plant Cell Tiss. Organ Cult. 89:159-168); insecticidal proteins from *Photorhabdus* sp. and *Xenorhabdus* sp. (Hinchliffe, et al., (2010) The Open Toxicology Journal, 3:101-118 and Morgan, et al., (2001) Applied and Envir. Micro. 67:2062-2069); US Patent Number 6,048,838, and US Patent Number 6,379,946; a PIP-1 polypeptide of US Patent Publication US20140007292; an AfIP-1A and/or AfIP-1B polypeptide of US Patent Publication US20140033361; a PHI-4 polypeptide of US Serial Number 13/839702; a PIP-47 polypeptide of PCT Serial Number PCT/US14/51063; a PIP-72 polypeptide of PCT Serial Number; a PtIP-50 polypeptide and a PtIP-65 polypeptide of PCT Publication Number WO2015/120270; a PtIP-83 polypeptide of PCT Publication Number WO2015/120276 ; a PtIP-96 polypeptide of PCT Serial Number PCT/US15/55502; PCT/US14/55128 and δ-endotoxins including, but not limited to, the Cry1, Cry2, Cry3, Cry4, Cry5, Cry6, Cry7, Cry8, Cry9, Cry 10, Cry11, Cry12, Cry13, Cry14, Cry15, Cry16, Cry17, Cry18, Cry19, Cry20, Cry21, Cry22, Cry23, Cry24, Cry25, Cry26, Cry27, Cry 28, Cry 29, Cry 30, Cry31, Cry32, Cry33, Cry34, Cry35,Cry36, Cry37, Cry38, Cry39, Cry40, Cry41, Cry42, Cry43, Cry44, Cry45, Cry 46, Cry47, Cry49, Cry 51 and Cry55 classes of δ-endotoxin genes and the *B. thuringiensis* cytolytic Cyt1 and Cyt2 genes. Other Cry proteins are well known to one skilled in the art (see, Crickmore, et al., "Bacillus thuringiensis toxin nomenclature" (2011), at lifesci.sussex.ac.uk/home/Neil_Crickmore/Bt/ which can be accessed on the world-wide web using the "www" prefix). The insecticidal activity of Cry proteins is well known to one skilled in the art (for review, see, van Frannkenhuyzen, (2009) J. Invert. Path. 101:1-16). The use of Cry proteins as transgenic plant traits is well known to one skilled in the art and Cry-transgenic plants including but not limited to Cry1Ac, Cry1Ac+Cry2Ab, Cry1Ab, Cry1A.105, Cry1F, Cry1Fa2, Cry1F+Cry1Ac, Cry2Ab, Cry3A, mCry3A, Cry3Bb1, Cry34Ab1, Cry35Ab1, Vip3A, mCry3A, Cry9c and CBI-Bt have received regulatory approval (see, Sanahuja, (2011) Plant Biotech Journal 9:283-300 and the CERA (2010) GM Crop Database Center for Environmental Risk Assessment (CERA), ILSI Research Foundation, Washington D.C. at cera-gmc.org/index.php?action=gm_crop_database which can be accessed on the world-wide web using the "www" prefix). As used herein, a "non-Bt trait" refers to any insecticidal gene or trait in a plant derived or modified from a naturally occurring bacterial, plant, or animal, excluding any *Bacillus thurengiensis* strain. Non-Bt traits include, but are not limited to a RNAi or dsRNA traits, a *Psuedomonas-derived* trait, or a plant-derived trait.

In one aspect, the disclosure relates to the use of the entomopathogenic fungal strains disclosed herein encapsulated in an alginate bead with an RNAi trait comprising a silencing construct of one or more polynucleotides of interest resulting in suppression of one or more target pathogen, pest, or insect polypeptides. By "silencing element" is it intended to mean a polynucleotide which when contacted by or ingested by a pest, is capable of reducing or eliminating the level or expression of a target polynucleotide or the polypeptide encoded thereby. The silencing element employed can reduce or eliminate the expression level of the target sequence by influencing the level of the target RNA transcript or, alternatively, by influencing translation and thereby affecting the level of the encoded polypeptide. Silencing elements may include, but are not limited to, a sense suppression element, an antisense suppression element, a double stranded RNA, a siRNA, an amiRNA, a miRNA, or a hairpin suppression element.

In another aspect, the disclosure relates to the use of the entomopathogenic fungal strains disclosed herein encapsulated in an alginate bead with a composition comprising nucleic acid molecules including silencing elements for targeting the vacuolar ATPase H subunit, useful for controlling a coleopteran pest population and infestation as described in US Patent Application Publication 2012/0198586. PCT Publication WO 2012/055982 describes ribonucleic acid (RNA or double stranded RNA) that inhibits or down regulates the expression of a target gene that encodes: an insect ribosomal protein such as the ribosomal protein L19, the ribosomal protein L40 or the ribosomal protein S27A; an insect proteasome subunit such as the Rpn6 protein, the Pros 25, the Rpn2 protein, the proteasome beta 1 subunit protein or the Pros beta 2 protein; an insect β-coatomer of the COPI vesicle, the γ-coatomer of the COPI vesicle, the β'- coatomer protein or the ζ-coatomer of the COPI vesicle; an insect Tetraspanine 2 A protein which is a putative transmembrane domain protein; an insect protein belonging to the actin family such as Actin 5C; an insect ubiquitin-5E protein; an insect Sec23 protein which is a GTPase activator involved in intracellular protein transport; an insect crinkled protein which is an unconventional myosin which is involved in motor activity; an insect crooked neck protein which is involved in the regulation of nuclear alternative mRNA splicing; an insect vacuolar HT-ATPase G-subunit protein and an insect Tbp-1 such as Tat-binding protein. PCT publication WO 2007/035650 describes ribonucleic acid (RNA or double stranded RNA) that inhibits or down regulates the expression of a target gene that encodes Snf7. US Patent Application publication 2011/0054007 describes polynucleotide silencing elements targeting RPS10. US Patent Application publication 2014/0275208 and US2015/0257389 describe polynucleotide silencing elements targeting RyanR and PAT3. PCT publications WO 2016/060911, WO 2016/060912, WO 2016/060913, and WO 2016/060914 describe polynucleotide silencing elements targeting COPI coatomer subunit nucleic acid molecules that confer resistance to Coleopteran and Hemipteran pests. International Application Number PCT/US2016/037748 describes polynucleotide silencing elements targeting VgR, MAEL, NCLB, and BOULE that control Coloepteran insect pests. US Patent Application Publications 2012/029750, US 20120297501, and 2012/0322660 describe interfering ribonucleic acids (RNA or double stranded RNA) that functions upon uptake by an insect pest species to down-regulate expression of a target gene in said insect pest, wherein the RNA comprises at least one silencing element wherein the silencing element is a region of double-stranded RNA comprising annealed complementary strands, one strand of which comprises or consists of a sequence of nucleotides which is at least partially complementary to a target nucleotide sequence within the target gene. US Patent Application Publication 2012/0164205 describe potential targets for interfering double stranded ribonucleic acids for inhibiting invertebrate pests including: a Chd3 Homologous Sequence, a Beta-Tubulin Homologous Sequence, a 40 kDa V-ATPase Homologous Sequence, a EF1α Homologous Sequence, a 26S Proteosome Subunit p28 Homologous Sequence, a Juvenile Hormone Epoxide Hydrolase Homologous Sequence, a Swelling Dependent Chloride Channel Protein Homologous Sequence, a Glucose-6-Phosphate 1-Dehydrogenase Protein Homologous Sequence, an Act42A Protein Homologous Sequence, a ADP-Ribosylation Factor 1 Homologous Sequence, a Transcription Factor IIB Protein Homologous Sequence, a Chitinase Homologous Sequences, a Ubiquitin Conjugating Enzyme Homologous Sequence, a Glyceraldehyde-3-Phosphate Dehydrogenase Homologous Sequence, an Ubiquitin B Homologous Sequence, a Juvenile Hormone Esterase Homolog, and an Alpha Tubulin Homologous Sequence.

One aspect of the disclosure comprises an additional component, which may be a carrier, a filler, an adjuvant, a solubilizing agent, a suspending agent, a diluent, an oxygen scavenger, an antioxidant, an oxygen barrier, a water barrier, a moisture barrier, a food material, a food source, another microbe, an anti-contaminant agent, or combinations thereof.

In another aspect of the disclosure, the additional component(s) may be required for the application to which the strain encapsulated in an alginate bead or composition containing such is to be utilized. For example, if the strain encapsulated in an alginate bead or composition containing such is to be utilized on, or in, an agricultural product, the additional component(s) may be an agriculturally acceptable carrier, excipient, or diluent. Likewise, if the strain encapsulated in an alginate bead or composition containing such is to be utilized on, or in, a foodstuff the additional component(s) may be an edible carrier, excipient or diluent.

In one aspect, the one or more additional component(s) is a carrier, excipient, or diluent.

"Carriers" or "vehicles" mean materials suitable for compound administration and include any such material known in the art such as, for example, any liquid, gel, solvent, liquid diluent, or solubilizer which is non-toxic and does not interact with any components of the composition in a deleterious manner.

Examples of nutritionally acceptable carriers include, for example, water, salt solutions, alcohol, silicone, waxes, petroleum jelly, vegetable oils, polyethylene glycols, propylene glycol, liposomes, sugars, gelatin, lactose, amylose, starches, magnesium stearate, talc, surfactants, silicic acid, viscous paraffin, perfume oil, fatty acid monoglycerides and diglycerides, petroethral fatty acid esters, hydroxymethyl-cellulose, and polyvinylpyrrolidone.

Examples of excipients include but are not limited to: microcrystalline cellulose and other celluloses, lactose, sodium citrate, calcium carbonate, dibasic calcium phosphate, glycine, starch, milk sugar, and high molecular weight polyethylene glycols.

Examples of diluents include but are not limited to: water, ethanol, propylene glycol and glycerin, and combinations thereof.

Additional components may be used simultaneously with an entomopathogenic fungal strain encapsulated in an alginate bead and/or a composition containing such disclosed herein (e.g. when they are in admixture together or even when they are delivered by different routes) or sequentially (e.g. they may be delivered by different routes).

An entomopathogenic fungal strain encapsulated in an alginate bead and/or a composition containing such disclosed herein and/or its diluent may also contain chelating agents such as EDTA, citric acid, tartaric acid, etc. Moreover, an entomopathogenic fungal strain encapsulated in an alginate bead and/or a composition containing such disclosed herein and/or its diluent may contain active agents selected from fatty acids esters, such as mono-and diglycerides, non-ionic surfactants, such as polysorbates, phospholipids, etc. An entomopathogenic fungal strain encapsulated in an alginate bead and/or a composition containing such disclosed herein and/or its diluent may also contain emulsifiers, which may enhance the stability of an entomopathogenic fungal strain and/or a composition, especially after dilution.

An entomopathogenic fungal strain encapsulated in an alginate bead and/or a composition containing such disclosed herein may be used in any suitable form, whether when used alone or when present in a composition. An entomopathogenic fungal strain encapsulated in an alginate bead and/or a composition containing such disclosed herein may be formulated in any suitable way to ensure that the composition comprises an active entomopathogenic fungal strain.

An entomopathogenic fungal strain encapsulated in an alginate bead and/or compositions containing such may be in the form of a dry powder that can be sprinkled on or mixed in with a product. Entomopathogenic fungal strains encapsulated in an alginate bead and/or compositions containing such of the aspects disclosed herein in the form of a dry powder may include additional additives such as clay, microcrystalline cellulose, gum tragacanth, gelatin, starch, lactose, Primojel^{®}, or corn starch.

In one aspect, the compositions disclosed herein can comprise concentrated, dried propagules, such as microsclerotia encapsulated in an alginate bead, from the entomopathogenic fungal strains disclosed herein. In one aspect of the disclosure, the concentrated, dried microsclerotia encapsulated in an alginate bead can be in the range of 0.0001mg to 5mg. In one aspect of the disclosure, the concentrated, dried microsclerotia encapsulated in an alginate bead can be in the range of 1 × 10² to 1 × 10¹⁴ CFU/mg.

In one aspect of the disclosure, entomopathogenic fungal strains encapsulated in an alginate bead and/or compositions containing such disclosed herein can be applied wet, partially desiccated, completely desiccated, or in any other form.

In at least some aspects of the disclosure, entomopathogenic fungal strains encapsulated in an alginate bead and/or compositions containing such can be freeze-dried or lyophilized. In at least some aspects of the disclosure, entomopathogenic fungal strains encapsulated in an alginate bead and/or compositions containing such can be mixed with a carrier. The carrier includes but is not limited to whey, maltodextrin, sucrose, dextrose, limestone (calcium carbonate), rice hulls, yeast culture, dried starch, clay, and sodium silico aluminate. In one aspect of the disclosure, the strains encapsulated in an alginate bead or compositions containing such can be in the form of a pellet or a granule.

An entomopathogenic fungal strain encapsulated in an alginate bead and/or a composition containing such described herein can be added to one or more carrier. Where used, the carrier(s) and the strains encapsulated in an alginate bead can be added to a ribbon or paddle mixer and mixed for about 15 minutes, although the timing can be increased or decreased. The components are blended such that a uniform mixture of the strain encapsulated in an alginate bead and carrier(s) is produced. The final product is preferably a dry, flowable powder.

In an aspect of the disclosure, an entomopathogenic fungal strain encapsulated in an alginate bead and/or compositions containing such may be formulated as a liquid, a dry powder, or a granule. The dry powder or granules may be prepared by means known to those skilled in the art, such as, in top-spray fluid bed coater, in a bottom spray Wurster, or by drum granulation (e.g. high sheer granulation), extrusion, pan coating or in a micro-ingredients mixer.

In another aspect of the disclosure, entomopathogenic fungal strains encapsulated in an alginate bead and/or compositions containing such may be provided as a spray-dried or freeze-dried powder.

In yet another aspect of the disclosure, the entomopathogenic fungal strains encapsulated in an alginate bead and/or compositions containing such are in a liquid formulation. Such liquid consumption may contain one or more of the following: a buffer, salt, sorbitol, and/or glycerol.

In one aspect of the disclosure, the entomopathogenic fungal strains encapsulated in an alginate bead and/or compositions containing such disclosed herein may be formulated with at least one physiologically acceptable carrier selected from at least one of maltodextrin, calcined (illite) clay, limestone (calcium carbonate), cyclodextrin, wheat or a wheat component, sucrose, starch, Na₂SO₄, talc, PVA, sorbitol, benzoate, sorbiate, glycerol, sucrose, propylene glycol, 1,3-propane diol, glucose, parabens, sodium chloride, citrate, acetate, phosphate, calcium, metabisulfite, formate and mixtures thereof.

In one aspect of the disclosure, the entomopathogenic fungal strains encapsulated in an alginate bead and/or compositions containing such disclosed herein are formulated to improve fungal propagule stability and as a way to protect the fungal propagules from seed applied fungicides. In one aspect of the disclosure, the encapsulation technology may comprise a bead polymer for timed release of fungal propagules, such as spores, over time. In one aspect of the disclosure, the encapsulated entomopathogenic fungal strains and/or compositions containing such may be applied in a separate application of beads in-furrow to the seeds. In another aspect of the disclosure, the encapsulated entomopathogenic fungal strains and/or compositions containing such may be co-applied along with seeds simultaneously.

A coating agent usable for the sustained release microparticles of an encapsulation aspect of the disclosure may be a substance which is useful for coating the microgranular form with the substance to be supported thereon. Any coating agent which can form a coating permeable for the supported substance may be used in general, without any particular limitation. For example, higher saturated fatty acid, wax, thermoplastic resin, and thermosetting resin may be used.

Examples of useful higher saturated fatty acid include stearic acid, zinc stearate, stearic acid amide and ethylenebis-stearic acid amide; those of wax include synthetic waxes such as polyethylene wax, carbon wax, Hoechst wax, and fatty acid ester; natural waxes such as carnauba wax, bees wax and Japan wax; and petroleum waxes such as paraffin wax and petrolatum. Examples of thermoplastic resin include polyolefins such as polyethylene, polypropylene, polybutene and polystyrene; vinyl polymers such as polyvinyl acetate, polyvinyl alcohol, polyvinyl chloride, polyvinylidene chloride, polyacrylic acid, polymethacrylic acid, polyacrylate and polymethacrylate; diene polymers such as butadiene polymer, isoprene polymer, chloroprene polymer, butadiene-styrene copolymer, ethylene-propylene-diene copolymer, styrene-isoprene copolymer, MMA-butadiene copolymer and acrylonitrile-butadiene copolymer; polyolefin copolymers such as ethylene-propylene copolymer, butene-ethylene copolymer, butene-propylene copolymer, ethylene-vinyl acetate copolymer, ethylene-acrylic acid copolymer, styreneacrylic acid copolymer, ethylene-methacrylic acid copolymer, ethylene-methacrylic ester copolymer, ethylene-carbon monoxide copolymer, ethylene-vinyl acetate-carbon monoxide copolymer, ethylene-vinyl acetate-vinyl chloride copolymer and ethylene-vinyl acetate-acrylic copolymer; and vinyl chloride copolymers such as vinyl chloride-vinyl acetate copolymer and vinylidene chloride-vinyl chloride copolymer. Examples of thermosetting resin include polyurethane resin, epoxy resin, alkyd resin, unsaturated polyester resin, phenolic resin, urea-melamine resin, urea resin and silicone resin. Of those, thermoplastic acrylic ester resin, butadienestyrene copolymer resin, thermosetting polyurethane resin and epoxy resin are preferred, and among the preferred resins, particularly thermosetting polyurethane resin is preferred. These coating agents can be used either singly or in combination of two or more kinds.

In one aspect of the disclosure, the entomopathogenic fungal strains encapsulated in an alginate bead, and/or compositions containing such may include a seed, a part of a seed, a plant, or a plant part.

All plants, plant parts, seeds or soil can be treated in accordance with the entomopathogenic fungal strains encapsulated in an alginate bead, compositions containing such, and methods disclosed herein. The compositions disclosed herein can include a plant, a plant part, a seed, a seed part, or soil. The entomopathogenic fungal strains encapsulated in an alginate bead, compositions containing such, and methods disclosed herein can be applied to the seed, the plant or plant parts, the fruit, or the soil in which the plants grow.

An aspect of the disclosure relates to a process for preparing entomopathogenic fungal propagules from liquid culture comprising: (a) mixing entomopathogenic fungal propagules with a polymeric encapsulating material, (b) forming hydrogel beads comprising entomopathogenic fungal propagules and encapsulant, (c) optionally drying hydrogel beads comprising entomopathogenic fungal propagules and optionally (d) size-reduction of hydrogel beads comprising entomopathogenic fungal propagules.

An aspect of the disclosure relates to a method for reducing plant pathogen, pest, or insect damage to a plant or plant part comprising: (a) treating a seed with an entomopathogenic fungal strain encapsulated in an alginate bead or composition containing such disclosed herein prior to planting. In another aspect of the disclosure, the method further comprises: (b) treating a plant part obtained from the seed with an entomopathogenic fungal strain encapsulated in an alginate bead or composition containing such disclosed herein. The entomopathogenic fungal strain encapsulated in an alginate bead or composition containing such used in step (a) may be the same or different than the entomopathogenic fungal strain encapsulated in an alginate bead or composition containing such used in step (b).

An aspect of the disclosure relates to a method for reducing plant pathogen, pest, or insect damage to a plant or plant part comprising: (a) treating the soil surrounding a seed or plant with an entomopathogenic fungal strain encapsulated in an alginate bead or composition containing such. In another aspect of the disclosure, the method further comprises: (b) treating a plant part with an entomopathogenic fungal strain encapsulated in an alginate bead or composition containing such disclosed herein. The entomopathogenic fungal strain encapsulated in an alginate bead or composition containing such used in step (a) may be the same or different than the entomopathogenic fungal strain encapsulated in an alginate bead or composition containing such used in step (b).

An aspect of the disclosure relates to a method for reducing plant pathogen, pest, or insect damage to a plant or plant part comprising: (a) treating a seed prior to planting with an entomopathogenic fungal strain encapsulated in an alginate bead or composition containing such disclosed herein. In another aspect of the disclosure, the method further comprises: (b) treating the soil surrounding the seed or plant with an entomopathogenic fungal strain encapsulated in an alginate bead or composition containing such disclosed herein. In still another aspect of the disclosure, the method further comprises: (c) treating a plant part of a plant produced from the seed with an entomopathogenic fungal strain encapsulated in an alginate bead or composition containing such disclosed herein. The entomopathogenic fungal strain encapsulated in an alginate bead or composition containing such used in step (a) may be the same or different than the entomopathogenic fungal strain encapsulated in an alginate bead or composition containing such used in step (b). The entomopathogenic fungal strain encapsulated in an alginate bead or composition containing such used in step (a) may be the same or different than the entomopathogenic fungal strain encapsulated in an alginate bead or composition containing such used in step (c). The entomopathogenic fungal strain encapsulated in an alginate bead or composition containing such used in step (b) may be the same or different than the entomopathogenic fungal strain encapsulated in an alginate bead or composition containing such used in step (c).

In one aspect of the disclosure, wild plant species and plant cultivars, or those obtained by conventional biological breeding, such as crossing or protoplast fusion, and parts thereof, can be treated with one or more entomopathogenic fungal strains encapsulated in an alginate bead, compositions containing such and methods disclosed herein. In another aspect of the disclosure, transgenic plants and plant cultivars obtained by genetic engineering, and plant parts thereof, are treated with one or more entomopathogenic fungal strains encapsulated in an alginate bead, compositions containing such and methods disclosed herein.

In another aspect of the disclosure, plants or plant cultivars (obtained by plant biotechnology methods such as genetic engineering) that may be treated according to the encapsulated strains, compositions containing such and methods disclosed herein are herbicide-tolerant plants, i.e. plants made tolerant to one or more given herbicides. Such plants can be obtained either by genetic modification, or by selection of plants containing a mutation imparting such herbicide tolerance. Herbicide-resistant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshilcimate-3-phosphate synthase (EPSPS).

Seeds, plants or plant cultivars (obtained by plant biotechnology methods such as genetic engineering) that may also be treated according to the aspects disclosed herein are insect-resistant genetically modified plants (or transgenic plants), *i.e.* plants made resistant to attack by certain target insects. Such plants can be obtained by genetic transformation, or by selection of plants containing a mutation imparting such insect resistance.

In another aspect of the disclosure, seeds, plants or plant cultivars (obtained by plant biotechnology methods such as genetic engineering) that may be treated according to the disclosure are tolerant to abiotic stresses. Such plants can be obtained by genetic transformation, or by selection of plants containing a mutation imparting such stress resistance.

In another aspect of the disclosure, seeds, plants or plant cultivars (obtained by plant biotechnology methods such as genetic engineering) that may be treated according to the disclosure are conventionally bred, by mutagenesis, or genetically engineered to contain a combination or stack of valuable traits, including but not limited to, herbicide tolerance, insect resistance, and abiotic stress tolerance. The aspects disclosed herein also apply to plant varieties which will be developed, or marketed, in the future and which have these genetic traits or traits to be developed in the future.

As used herein, applying an encapsulated entomopathogenic fungal strain, an alginate bead encapsulating fungal entomopathogenic microsclerotia, or a composition containing such to a seed, a plant, or plant part includes contacting, spraying, coating, misting, and/or applying to the seed, plant, or plant part directly and/or indirectly with the encapsulated entomopathogenic fungal strain, the alginate bead encapsulating fungal entomopathogenic microsclerotia or the composition containing such. In one aspect of the disclosure, an encapsulated entomopathogenic fungal strain, an alginate bead encapsulating fungal entomopathogenic microsclerotia, or a composition containing such may be directly applied as a spray, a rinse, coating, or a powder, or any combination thereof. A contacting step may occur while a seed, a plant or a plant part is being grown, while a plant or a plant part is being fertilized, while a plant or a plant part is being harvested, after a plant or a plant part has been harvested, while a plant or a plant part is being processed, while a plant or a plant part is being packaged, or while a plant or a plant part is being stored in a warehouse or on a shelf in a store.

As used herein, a spray refers to a mist of liquid particles that contain an encapsulated entomopathogenic fungal strain, an alginate bead encapsulating fungal entomopathogenic microsclerotia, or a composition containing such of the present disclosure. In one aspect of the disclosure, a spray may be applied to a seed, plant, or plant part while a plant or plant part is being grown. In another aspect, a spray may be applied to a seed, plant, or plant part while a seed, plant, or plant part is being fertilized. In another aspect, a spray may be applied to a seed, plant, or plant part while a seed, plant, or plant part is being harvested. In another aspect, a spray may be applied to a seed, plant, or plant part after a seed, plant, or plant part has been harvested. In another aspect, a spray may be applied to a seed, plant, or plant part while a plant or plant part is being processed. In another aspect, a spray may be applied to a seed, plant, or plant part while a seed, plant, or plant part is being packaged. In another aspect, a spray may be applied to a seed, plant, or plant part while a seed, plant, or plant part is being stored.

In another aspect of the disclosure, an encapsulated entomopathogenic fungal strain, an alginate bead encapsulating fungal entomopathogenic microsclerotia, or a composition containing such disclosed herein may be applied directly to a seed, plant, or plant part as a rinse. As used herein, a rinse is a liquid containing an entomopathogenic fungal strain or composition disclosed herein. Such a rinse may be poured over a seed, plant or plant part. A plant or plant part may also be immersed or submerged in the rinse, then removed and allowed to dry.

In another aspect of the disclosure, an encapsulated entomopathogenic fungal strain, an alginate bead encapsulating fungal entomopathogenic microsclerotia, or a composition containing such may be applied to a seed, plant, or plant part and may cover 50% of the surface area of a plant material. In another aspect of the disclosure, an encapsulated entomopathogenic fungal strain, an alginate bead encapsulating fungal entomopathogenic microsclerotia, or a composition containing such may be applied to a plant or plant part and may cover a percentage of the surface area of a plant material from 50% to about 95%, from 60% to about 95%, from 70% to about 95%, from 80% to about 95%, and from 90% to about 95%. In another aspect of the disclosure, an encapsulated entomopathogenic fungal strain, an alginate bead encapsulating fungal entomopathogenic microsclerotia, or a composition containing such disclosed herein may be applied to the environment of a seed, a plant or a plant part and may cover 50% of the surface area of the environment of a seed, a plant or a plant part. In another aspect of the disclosure, an encapsulated entomopathogenic fungal strain, an alginate bead encapsulating fungal entomopathogenic microsclerotia, or a composition containing such may be applied to the environment of a seed, a plant or a plant part and may cover a percentage of the surface area of the environment of a seed, a plant or a plant part from 50% to about 95%, from 60% to about 95%, from 70% to about 95%, from 80% to about 95%, and from 90% to about 95%.

In another aspect, an encapsulated entomopathogenic fungal strain, an alginate bead encapsulating fungal entomopathogenic microsclerotia, or a composition containing such may cover from about 20% to about 30%, from about 30% to about 40%, from about 40% to about 50%, from about 50% to about 60%, from about 60% to about 70%, from about 70% to about 80%, from about 80% to about 90%, from about 90% to about 95%, from about 95% to about 98%, from about 98% to about 99% or 100% of the surface area of a seed, a plant or a plant part or the environment of a seed, a plant or a plant part.

In another aspect of the disclosure, an encapsulated entomopathogenic fungal strain, an alginate bead encapsulating fungal entomopathogenic microsclerotia, or a composition containing such disclosed herein may be applied directly to a seed, a plant or a plant part or the environment of a seed, a plant or a plant part as a powder. As used herein, a powder is a dry or nearly dry bulk solid composed of a large number of very fine particles that may flow freely when shaken or tilted. A dry or nearly dry powder composition disclosed herein preferably contains a low percentage of water, such as, for example, in various aspects, less than 5%, less than 2.5%, or less than 1% by weight.

In another aspect of the disclosure, an encapsulated entomopathogenic fungal strain, an alginate bead encapsulating fungal entomopathogenic microsclerotia, or a composition containing such can be applied indirectly to a seed, a plant or a plant part or the environment of a seed, a plant or a plant part. For example, a seed, plant, or plant part having an entomopathogenic fungal strain or composition already applied may be touching a second seed, plant, or plant part so that an encapsulated entomopathogenic fungal strain, an alginate bead encapsulating fungal entomopathogenic microsclerotia, or a composition containing such rubs off on a second seed, plant, or plant part. In a further aspect, an encapsulated entomopathogenic fungal strain, an alginate bead encapsulating fungal entomopathogenic microsclerotia, or a composition containing such may be applied using an applicator. In various aspects, an applicator may include, but is not limited to, a syringe, a sponge, a paper towel, or a cloth, or any combination thereof.

A contacting step may occur while a plant material is being grown, while a seed, plant, or plant part is being fertilized, while a plant or plant part is being harvested, after a seed, plant, or plant part has been harvested, while a plant or plant part is being processed, while a plant or plant part is being packaged, or while a plant or plant part is being stored in a warehouse.

In another aspect of the disclosure, an encapsulated entomopathogenic fungal strain, an alginate bead encapsulating fungal entomopathogenic microsclerotia, or a composition containing such disclosed herein may be a colloidal dispersion. A colloidal dispersion is a type of chemical mixture where one substance is dispersed evenly throughout another. Particles of the dispersed substance are only suspended in the mixture, unlike a solution, where they are completely dissolved within. This occurs because the particles in a colloidal dispersion are larger than in a solution - small enough to be dispersed evenly and maintain a homogenous appearance, but large enough to scatter light and not dissolve. Colloidal dispersions are an intermediate between homogeneous and heterogeneous mixtures and are sometimes classified as either "homogeneous" or "heterogeneous" based upon their appearance.

In one aspect of the disclosure, the encapsulated entomopathogenic fungal strains, the alginate bead encapsulating fungal entomopathogenic microsclerotia, compositions containing such, and methods disclosed herein are suitable for use with a seed. In another aspect of the disclosure, the encapsulated entomopathogenic fungal strains, the alginate bead encapsulating fungal entomopathogenic microsclerotia, compositions containing such, and methods disclosed herein are suitable for use with a seed of one or more of any of the plants recited previously.

In still another aspect of the disclosure, encapsulated entomopathogenic fungal strains, alginate beads encapsulating fungal entomopathogenic microsclerotia, compositions containing such and methods disclosed herein can be used to treat transgenic or genetically modified seed. The heterologous gene in transgenic seed can originate, for example, from microorganisms of the species *Bacillus, Rhizobium, Pseudomonas, Serratia, Trichoderma, Clavibacter, Glomus* or *Gliocladium.*

In one aspect of the disclosure, a seed is treated in a state in which it is sufficiently stable so that the treatment does not cause any damage. In general, treatment of a seed may take place at any point in time between harvesting and sowing. In one aspect of the disclosure, a seed used is separated from the plant and freed from cobs, shells, stalks, coats, hairs or the flesh of the fruits. Thus, it is possible to use, for example, a seed which has been harvested, cleaned and dried. Alternatively, it is also possible to use a seed which, after drying, has been treated, for example, with water and then dried again.

In one aspect of the disclosure, seed is treated with the encapsulated entomopathogenic fungal strains, alginate beads encapsulating fungal entomopathogenic microsclerotia, compositions containing such, and methods disclosed herein in such a way that the germination of a seed is not adversely affected, or that the resulting plant is not damaged.

In one aspect of the disclosure, encapsulated entomopathogenic fungal strains, alginate beads encapsulating fungal entomopathogenic microsclerotia, and compositions containing such disclosed herein can be applied directly to a seed. For example, encapsulated entomopathogenic fungal strains, alginate beads encapsulating fungal entomopathogenic microsclerotia, compositions containing such and methods disclosed herein can be applied without additional components and without having been diluted.

In another aspect of the disclosure, encapsulated entomopathogenic fungal strains, alginate beads encapsulating fungal entomopathogenic microsclerotia, and compositions containing such disclosed herein are applied to a seed in the form of a suitable formulation. Suitable formulations and methods for the treatment of seed are known to the person skilled in the art and are described, for example, in the following documents: US 4,272,417 A, US 4,245,432 A, US 4,808,430 A, US 5,876,739 A, US 2003/0176428 A1, WO 2002/080675 A1, WO 2002/028186 A2.

The encapsulated entomopathogenic fungal strains, alginate beads encapsulating fungal entomopathogenic microsclerotia, and compositions containing such disclosed herein can be converted into customary seed dressing formulations, such as solutions, emulsions, suspensions, powders, foams, slurries or other coating materials for seed, and also ULV formulations. These formulations are prepared in a known manner by mixing the encapsulated entomopathogenic fungal strains, alginate beads encapsulating fungal entomopathogenic microsclerotia, and compositions containing such disclosed herein with customary additives, such as, for example, customary extenders and also solvents or diluents, colorants, wetting agents, dispersants, emulsifiers, defoamers, preservatives, secondary thickeners, adhesives, gibberellins and water as well.

In another aspect of the disclosure, suitable colorants that may be present in a seed dressing formulation include all colorants customary for such purposes. Use may be made both of pigments, of sparing solubility in water, and of dyes, which are soluble in water. Examples that may be mentioned include the colorants known under the designations Rhodamine B, C.I. Pigment Red 112, and C.I. Solvent Red 1.

In another aspect of the disclosure, suitable wetting agents that may be present in a seed dressing formulation include all substances that promote wetting and are customary in the formulation of active agrochemical substances. With preference it is possible to use alkylnaphthalene-sulphonates, such as diisopropyl- or diisobutylnaphthalene-sulphonates.

In still another aspect of the disclosure, suitable dispersants and/or emulsifiers that may be present in a seed dressing formulation include all nonionic, anionic, and cationic dispersants that are customary in the formulation of active agrochemical substances. In one aspect of the disclosure, nonionic or anionic dispersants or mixtures of nonionic or anionic dispersants can be used. In one aspect of the disclosure, nonionic dispersants include but are not limited to ethylene oxide-propylene oxide block polymers, alkylphenol polyglycol ethers, and tristyrylphenol polyglycol ethers, and their phosphated or sulphated derivatives.

In still another aspect of the disclosure, defoamers that may be present in a seed dressing formulation to be used according to the aspects of the disclosure include all foam-inhibiting compounds that are customary in the formulation of agrochemically active compounds including but not limited to silicone defoamers, magnesium stearate, silicone emulsions, long-chain alcohols, fatty acids and their salts and also organofluorine compounds and mixtures thereof.

In still another aspect of the disclosure, secondary thickeners that may be present in a seed dressing formulation include all compounds which can be used for such purposes in agrochemical compositions, including but not limited to cellulose derivatives, acrylic acid derivatives, polysaccharides, such as xanthan gum or Veegum, modified clays, phyllosilicates, such as attapulgite and bentonite, and also finely divided silicic acids.

Suitable adhesives that may be present in a seed dressing formulation to be used may include all customary binders which can be used in seed dressings. Polyvinylpyrrolidone, polyvinylpolypyrrolidone, polyvinyl acetate, polyvinyl alcohol and tylose may be mentioned as being preferred.

In yet another aspect of the disclosure, seed dressing formulations may be used directly or after dilution with water beforehand to treat seed of any of a very wide variety of types. The seed dressing formulations or their dilute preparations may also be used to dress seed of transgenic plants. In this context, synergistic effects may also arise in interaction with the substances formed by expression.

Suitable mixing equipment for treating seed with a seed dressing formulation or the preparations prepared from them by adding water includes all mixing equipment that can commonly be used for dressing. The specific procedure adopted when dressing comprises introducing the seed into a mixer, adding the particular desired amount of seed dressing formulation, either as it is or following dilution with water beforehand, and carrying out mixing until the formulation is uniformly distributed on the seed. Optionally, a drying operation follows.

In various aspects of the disclosure, one or more encapsulated entomopathogenic fungal strains, alginate beads encapsulating fungal entomopathogenic microsclerotia, compositions and/or formulations containing such can be added to the plant, plant part, and/or seed at a rate of about 10 to 1 × 10¹⁴ colony forming units (cfu) per seed, including about 1 × 10³ cfu/seed, or about 1 × 10⁴ cfu/seed, 1 × 10⁵ cfu/seed, or about 1 × 10⁶cfu/seed, or about 1 × 10⁷ cfu/seed, or about 1 × 10⁸ cfu/seed, or about 1 × 10⁹ cfu/seed, or about 1 × 10¹⁰ cfu/seed, or about 1 × 10¹¹ cfu/seed, or about 1 × 10¹² cfu/seed, or about 1 × 10¹³ cfu/seed including about 1 × 10³ to 1 × 10⁸ cfu/seed about 1 × 10³ to 1 × 10⁷ cfu/seed, about 1 × 10³ to 1 × 10⁵ cfu/seed, about 1 × 10³ to 1 × 10⁶ cfu/seed, about 1 × 10³ to 1 × 10⁴ cfu/seed, about 1 × 10³ to 1 × 10⁹ cfu/seed, about 1 × 10³ to 1 × 10¹⁰ cfu/seed, about 1 × 10³ to 1 × 10¹¹ cfu/seed, about 1 × 10³ to 1 × 10¹² cfu/seed, about 1 × 10³ to 1 × 10¹³ cfu/seed, about 1 × 10⁴ to 1 × 10⁸ cfu/seed about 1 × 10⁴ to 1 × 10⁷ cfu/seed, about 1 × 10⁴ to 1 × 10⁵ cfu/seed, about 1 × 10⁴ to 1 × 10⁶ cfu/seed, about 1 × 10⁴ to 1 × 10⁹ cfu/seed, about 1 × 10⁴ to 1 × 10¹⁰ cfu/seed, about 1 × 10¹¹ to 1 × 10⁹ cfu/seed, about 1 × 10⁴ to 1 × 10¹² cfu/seed about 1 × 10⁴ to 1 × 10¹³ cfu/seed, about 1 × 10⁵ to 1 × 10⁷ cfu/per seed, about 1 × 10⁵ to 1 × 10⁶ cfu/per seed, about 1 × 10⁵ to 1 × 10⁸ cfu/per seed, about 1 × 10⁵ to 1 × 10⁹ cfu/per seed, about 1 × 10⁵ to 1 × 10¹⁰ cfu/per seed, about 1 × 10⁵ to 1 × 10¹¹ cfu/per seed, about 1 × 10⁵ to 1 × 10¹² cfu/per seed, about 1 × 10⁵ to 1 × 10¹³ cfu/per seed, about 1 × 10⁶ to 1 × 10⁸ cfu/per seed, about 1 × 10⁶ to 1 × 10⁷ cfu/per seed, about 1 × 10⁶ to 1 × 10⁹ cfu/per seed, about 1 × 10⁶ to 1 × 10¹⁰ cfu/per seed, about 1 × 10⁶ to 1 × 10¹¹ cfu/per seed, about 1 × 10⁶ to 1 × 10¹² cfu/per seed, about 1 × 10⁶ to 1 × 10¹³ cfu/per seed, about 1 × 10⁷ to 1 × 10⁸ cfu/per seed, about 1 × 10⁷ to 1 × 10⁹ cfu/per seed, about 1 × 10⁷ to 1 × 10¹⁰ cfu/per seed, about 1 × 10⁷ to 1 × 10¹¹ cfu/per seed, about 1 × 10⁷ to 1 × 10¹² cfu/per seed, about 1 × 10⁷ to 1 × 10¹³ cfu/per seed, about 1 × 10⁸ to 1 × 10⁹ cfu/per seed, about 1 × 10⁸ to 1 × 10¹⁰ cfu/per seed, about 1 × 10⁸ to 1 × 10¹¹ cfu/per seed, about 1 × 10⁸ to 1 × 10¹² cfu/per seed, about 1 × 10⁸ to 1 × 10¹³ cfu/per seed, about 1 × 10⁹ to 1 × 10¹⁰ cfu/per seed, about 1 × 10⁹ to 1 × 10¹¹ cfu/per seed, about 1 × 10⁹ to 1 × 10¹² cfu/per seed, about 1 × 10⁹ to 1 × 10¹³ cfu/per seed, about 1 × 10¹⁰ to 1 × 10¹¹ cfu/per seed, about 1 × 10¹⁰ to 1 × 10¹² cfu/per seed, about 1 × 10¹⁰ to 1 × 10¹³ cfu/per seed, about 1 × 10¹¹¹ to 1 × 10¹² cfu/per seed, about 1 × 10¹¹ to 1 × 10¹³ cfu/per seed, and about 1 × 10¹² to 1 × 10¹³ cfu/per seed. As used herein, the term "colony forming unit" or "cfu" is a unit containing entomopathogen fungal structures capable of growing and producing a colony in favorable conditions. The cfu count serves as an estimate of the number of viable structures or cells in a sample.

In one aspect of the disclosure, encapsulated entomopathogenic fungal strains, alginate beads encapsulating fungal entomopathogenic microsclerotia, and compositions containing such can be formulated as a liquid seed treatment. The seed treatment comprises at least one encapsulated entomopathogenic fungal strain, alginate bead encapsulating fungal entomopathogenic microsclerotia, or a composition containing such. Seeds may be substantially uniformly coated with one or more layers of an encapsulated entomopathogenic fungus, an alginate bead encapsulating fungal entomopathogenic microsclerotia, or a composition containing such, using conventional methods of mixing, spraying or a combination thereof. Application may be done using equipment that accurately, safely, and efficiently applies seed treatment products to seeds. Such equipment uses various types of coating technology such as rotary coaters, drum coaters, fluidized bed techniques, spouted beds, rotary mists or a combination thereof.

In one aspect of the disclosure, the application is done via either a spinning "atomizer" disk or a spray nozzle that evenly distributes the seed treatment onto the seed as it moves through the spray pattern. In yet another aspect of the disclosure, a seed is then mixed or tumbled for an additional period of time to achieve additional treatment distribution and drying. Seeds may be primed or unprimed before coating with the inventive compositions to increase the uniformity of germination and emergence. In an alternative aspect of the disclosure, a dry powder composition can be metered onto the moving seed.

In still another aspect of the disclosure, a seed may be coated via a continuous or batch coating process. In a continuous coating process, continuous flow equipment simultaneously meters both the seed flow and the seed treatment products. A slide gate, cone and orifice, seed wheel, or weight device (belt or diverter) regulates seed flow. Once the seed flow rate through treating equipment is determined, the flow rate of the seed treatment is calibrated to the seed flow rate in order to deliver the desired dose to the seed as it flows through the seed treating equipment. Additionally, a computer system may monitor the seed input to the coating machine, thereby maintaining a constant flow of the appropriate amount of seed.

In a batch coating process, batch treating equipment weighs out a prescribed amount of seed and places the seed into a closed treating chamber or bowl where the corresponding of seed treatment is then applied. The seed and seed treatment are then mixed to achieve a substantially uniform coating on each seed. This batch is then dumped out of the treating chamber in preparation for the treatment of the next batch. With computer control systems, this batch process is automated enabling it to continuously repeat the batch treating process.

A variety of additives can be added to a seed treatment. Binders can be added and include those composed preferably of an adhesive polymer that can be natural or synthetic without phytotoxic effect on the seed to be coated. A variety of colorants may be employed, including organic chromophores classified as nitroso, nitro, azo, including monoazo, bisazo, and polyazo, diphenylmethane, triarylmethane, xanthene, methane, acridine, thiazole, thiazine, indamine, indophenol, azine, oxazine, anthraquinone, and phthalocyanine. Other additives that can be added include trace nutrients such as salts of iron, manganese, boron, copper, cobalt, molybdenum, and zinc. A polymer or other dust control agent can be applied to retain the treatment on the seed surface.

Other conventional seed treatment additives include, but are not limited to, coating agents, wetting agents, buffering agents, and polysaccharides. At least one agriculturally acceptable carrier can be added to the seed treatment formulation such as water, solids or dry powders. The dry powders can be derived from a variety of materials such as wood barks, calcium carbonate, gypsum, vermiculite, talc, humus, activated charcoal, biochar, and various phosphorous compounds.

In one aspect of the disclosure, a seed coating comprises at least one filler, which is an organic or inorganic, natural or synthetic component with which the encapsulated entomopathogenic fungal strains, alginate beads encapsulating fungal entomopathogenic microsclerotia, and compositions containing such thereof are combined to facilitate its application onto the seed. In one aspect of the disclosure, the filler is an inert solid such as clays, natural or synthetic silicates, silica, resins, waxes, solid fertilizers (for example ammonium salts), natural soil minerals, such as kaolins, clays, talc, lime, quartz, attapulgite, montmorillonite, bentonite, or diatomaceous earths, or synthetic minerals, such as silica, alumina, or silicates, in particular aluminum or magnesium silicates.

In one aspect of the disclosure, the encapsulated entomopathogenic fungal strains, alginate beads encapsulating fungal entomopathogenic microsclerotia, and/or compositions containing such disclosed herein may be formulated to improve fungal spore stability and as a way to protect the fungal spores from seed applied fungicides. In one aspect of the disclosure, the encapsulation technology may comprise a bead polymer for timed release of fungal spores over time. In one aspect of the disclosure, the encapsulation technology may comprise a zeolite material. In one aspect of the disclosure, the encapsulated entomopathogenic fungal strains, alginate beads encapsulating fungal entomopathogenic microsclerotia, and/or compositions containing such may be applied in a separate application of beads in-furrow to the seeds. In another aspect of the disclosure, the encapsulated entomopathogenic fungal strains, alginate beads encapsulating fungal entomopathogenic microsclerotia, and/or compositions containing such may be co-applied along with seeds simultaneously.

Insect resistance management (IRM) is the term used to describe practices aimed at reducing the potential for insect pests to become resistant to an insect management tactic. Maintenance of *Bt* (*Bacillus thuringiensis*) derived pesticidal proteins, other pesticidal proteins, a chemical, an entomopathogenic biological agent, or other biologicals, IRM is of great importance because of the threat insect resistance poses to the future use of pesticidal plant-incorporated protectants and insecticidal trait technology as a whole. Specific IRM strategies, such as the refuge strategy, mitigate insect resistance to specific insecticidal proteins produced in corn, soybean, cotton, and other crops. However, such strategies result in portions of crops being left susceptible to one or more pests in order to ensure that non-resistant insects develop and become available to mate with any resistant pests produced in protected crops. Accordingly, from a farmer/producer's perspective, it is highly desirable to have as small a refuge as possible and yet still manage insect resistance, in order that the greatest yield be obtained while still maintaining the efficacy of the pest control method used, whether *Bt,* a different pesticidal protein, a chemical, an entomopathogenic biological agent or other biologicals, some other method, or combinations thereof.

An often used IRM strategy is the planting of a refuge (a portion of the total acreage using non-Bt/pesticidal trait seed), as it is commonly-believed that this will delay the development of insect resistance to pesticidal traits by maintaining insect susceptibility. The theoretical basis of the refuge strategy for delaying resistance hinges on the assumption that the frequency and recessiveness of insect resistance is inversely proportional to pest susceptibility; resistance will be rare and recessive only when pests are very susceptible to the toxin, and conversely resistance will be more frequent and less recessive when pests are not very susceptible. Furthermore, the strategy assumes that resistance to an insecticidal trait is recessive and is conferred by a single locus with two alleles resulting in three genotypes: susceptible homozygotes (SS), heterozygotes (RS), and resistant homozygotes (RR). It also assumes that there will be a low initial resistance allele frequency and that there will be extensive random mating between resistant and susceptible adults. Under ideal circumstances, only rare RR individuals will survive a pesticidal toxin produced by the crop. Both SS and RS individuals will be susceptible to the pesticidal toxin. A structured refuge is a non-Bt/insecticidal trait portion of a grower's field or set of fields that provides for the production of susceptible (SS) insects that may randomly mate with rare resistant (RR) insects surviving the insecticidal trait crop, which may be a *Bt* trait crop, to produce susceptible RS heterozygotes that will be killed by the *Bt*/insecticidal trait crop. An integrated refuge is a certain portion of randomly planted non-Bt/insecticidal trait portion of a grower's field or set of fields that provides for the production of susceptible (SS) insects that may randomly mate with rare resistant (RR) insects surviving the insecticidal trait crop to produce susceptible RS heterozygotes that will be killed by the pesticidal trait crop. Each refuge strategy will remove resistant (R) alleles from the insect populations and delay the evolution of resistance.

Another strategy to reduce the need for refuge is the pyramiding of traits with different modes of action against a target insect pest. For example, *Bt* toxins that have different modes of action pyramided in one transgenic plant are able to have reduced refuge requirements due to reduced resistance risk. Different modes of action in a pyramid combination also extends the durability of each trait, as resistance is slower to develop to each trait.

Currently, the size, placement, and management of the refuge are often considered critical to the success of refuge strategies to mitigate insect resistance to the *Bt*/pesticidal trait produced in corn, cotton, soybean, and other crops. Because of the decrease in yield in refuge planting areas, some farmers choose to eschew the refuge requirements, and others do not follow the size and/or placement requirements. These issues result in either no refuge or a less effective refuge, and a corresponding risk of the increase in the development of resistance pests.

Accordingly, there remains a need for methods for managing pest resistance in a plot of pest resistant crop plants. It would be useful to provide an improved method for the protection of plants, especially corn or other crop plants, from feeding damage by pests. It would be particularly useful if such a method would reduce the required application rate of conventional chemical pesticides, and also if it would limit the number of separate field operations that were required for crop planting and cultivation. In addition, it would be useful to have a method of deploying a biocontrol agent that increases the durability of an insecticidal trait or increases the efficacy of many resistance management strategies.

One aspect of the disclosure relates to a method of reducing or preventing the development of resistance to a plant insecticidal/pesticidal composition of a pest in a population comprising providing a plant protection composition, such as a *Bt* pesticidal protein, a transgenic pesticidal protein, other pesticidal proteins, chemical pesticides, or pesticidal biological entomopathogens to a seed, a plant, a plant part, or a planted area. Another aspect of the disclosure relates to a method of reducing or preventing the resistance to a plant insecticidal trait comprising providing a composition comprising a plant insecticidal trait and an encapsulated entomopathogenic fungal strain, alginate beads encapsulating fungal entomopathogenic microsclerotia, and compositions containing such described herein. A further aspect of the disclosure relates to a method of reducing or preventing the resistance to a plant *Coleopteran* insecticidal trait comprising providing a composition comprising a plant *Coleopteran* insecticidal trait and an encapsulated entomopathogenic fungal strain, an alginate bead encapsulating fungal entomopathogenic microsclerotia, and/or a composition containing such described herein. Another aspect of the disclosure relates to a method of reducing or preventing the resistance to a plant *Diabrotica virgifera virgifera* insecticidal trait comprising providing a plant *Diabrotica virgifera virgifera* insecticidal trait and an encapsulated entomopathogenic fungal strain, an alginate bead encapsulating fungal entomopathogenic microsclerotia, and/or a composition containing such described herein. In certain aspects of the disclosure, the insecticidal trait comprises a *Bt* trait, a non-*Bt* trait, or an RNAi trait.

A further aspect of the disclosure relates to a method of increasing the durability of plant pest compositions comprising providing a plant protection composition to a seed, a plant or planted area, and providing the encapsulated entomopathogenic fungal strains, the alginate beads encapsulating fungal entomopathogenic microsclerotia, compositions containing such, and/or methods described herein to the seed, plant, or planted area, wherein the entomopathogenic fungal strains, compositions, and/or methods described herein have a different mode of action than the plant protection composition.

In a still further aspect of the disclosure, the refuge required may be reduced or eliminated by the presence of encapsulated entomopathogenic fungal strains, alginate beads encapsulating fungal entomopathogenic microsclerotia, compositions containing such, and/or methods described herein applied to the non-refuge plants. In another aspect of the disclosure, the refuge may include the encapsulated entomopathogenic fungal strains, the alginate beads encapsulating fungal entomopathogenic microsclerotia, compositions containing such, and/or methods described herein as a spray, bait, or as a different mode of action.

In one aspect of the disclosure, a composition comprises an encapsulated fungal entomopathogen, an alginate bead encapsulating fungal entomopathogenic microsclerotia, compositions containing such and a non-*Bt* insecticidal trait that increases resistance to a pathogen, pest, or insect. In another aspect of the disclosure, the encapsulated fungal entomopathogen is selected from the group consisting of: *Metarhizium robertsii* 15013-1, *Metarhizium robertsii* 23013-3, and *Metarhizium anisopliae* 3213-1. In another aspect of the disclosure, the non-*Bt* insecticidal trait comprises a plant-derived insecticidal protein, a bacterial/archeal-derived insecticidal protein not from a *Bt* (such as a *Pseudomonas* insecticidal protein), an animal-derived insecticidal protein, or a silencing element. In another aspect of the disclosure, a composition comprising an encapsulated fungal entomopathogen and a non-*Bt* insecticidal trait increases durability of the non-*Bt* insecticidal trait. In another aspect of the disclosure, the non-*Bt* insecticidal trait comprises a PIP-72 polypeptide of PCT Serial Number PCT/US14/55128. In another aspect of the disclosure, the non-*Bt* insecticidal trait comprises a polynucleotide silencing elements targeting RyanR, HP2, or PAT3 (US Patent Application publication 2014/0275208 and US2015/0257389). In another aspect of the disclosure, the non-*Bt* insecticidal trait comprises a polynucleotide silencing elements targeting RyanR (US Patent Application publication 2014/0275208) and a PIP-72 polypeptide of PCT Serial Number PCT/US14/55128.

In a further aspect of the disclosure, a composition that increases resistance to a pathogen, a pest, or an insect comprises an encapsulated fungal entomopathogen, such as entomopathogenic fungal strain disclosed herein, and a *Bt* insecticidal trait that increases resistance to a pathogen, pest, or insect. A *Bt* insecticidal trait may have activity against *Coleopteran* plant pests, such as *Diabrotica virgifera virgifera.* The compositions disclosed herein may provide to a plant or plant part additive or synergistic resistance to a pathogen, pest, or insect plant in combination with a *Bt* insecticidal trait. In one aspect of the disclosure, a composition comprises an encapsulated fungal entomopathogen and a *Bt* insecticidal trait, wherein the *Bt* insecticidal trait comprises a Cry3B toxin disclosed in US Patent Numbers 8,101,826, 6,551,962, 6,586,365, 6,593,273, and PCT Publication WO 2000/011185, a mCry3B toxin disclosed in US Patent Numbers 8,269,069, and 8,513,492, a mCry3A toxin disclosed in US Patent Numbers 8,269,069, 7,276,583 and 8,759,620, or a Cry34/35 toxin disclosed in US Patent Numbers 7,309,785, 7,524,810, 7,985,893, 7,939,651 and 6,548,291, and transgenic events containing these *Bt* insecticidal toxins and other *Coleopteran* active *Bt* insecticidal traits for example, event MON863 disclosed in US Patent Number 7,705,216, event MIR604 disclosed in US Patent Number 8,884,102, event 5307 disclosed in US Patent Number 9,133,474, event DAS-59122 disclosed in US Patent Number 7,875,429, event DP-4114 disclosed in US Patent Number 8,575,434, event MON 87411 disclosed in US Published Patent Application Number 2013/0340111, and event MON88017 disclosed in US Patent Number 8,686,230.

The subject-matter of the appended claims is illustrated by following examples.

All publications, patents and patent applications mentioned in the specification indicate the level of those skilled in the art to which this disclosure pertains.

### Example 1. Production of Metarhizium reporter strain expressing ZsGreen (not part of the claimed invention)

An actively growing culture of *Metarhizium* strain 15013-1 in Sabourauds Dextrose medium was harvested by centrifugation after 44h at 26°C. The pellet was resuspended in a buffer (0.01M EGTA, 0.5M NaCl in 0.1M sodium citrate, pH 5.8) containing Lysing enzyme (Sigma L1412; 10g per L culture pelleted). After shaking at room temperature for 3h, the preparation was filtered twice through 4 layers sterile muslin, then through one layer of 25um Nylon membrane (Nytex). Protoplasts were pelleted by centrifugation (3000rpm, 10min), then washed twice by resuspension and recentrifugation in 20% sucrose, 50mM CaCl₂, 50mM Tris-Cl, pH 8.0.

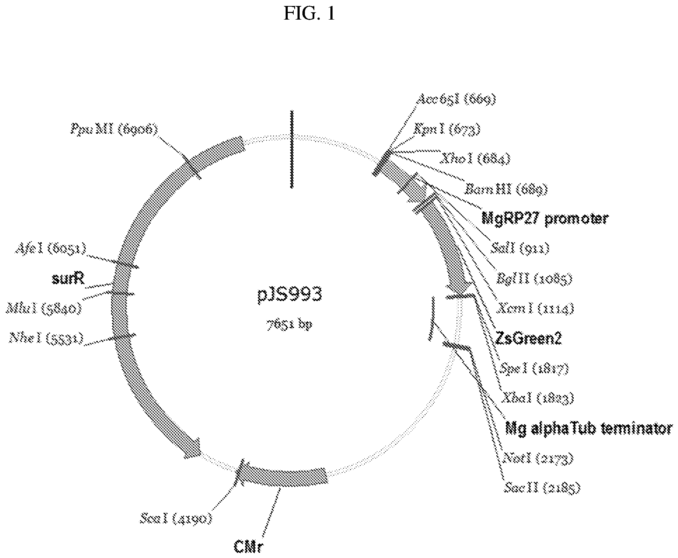

280µl protoplasts were transformed by adding 2µg plasmid pJS993 (FIG. 1) in 40µl TE buffer, and after incubation at room temperature for 20min, 2ml 40% PEG 3350 in 20% sucrose, 50mM CaCL2, 50mM Tris-Cl, pH8.0. After a further incubation at room temperature, 3ml 20% sucrose in Aspergillus minimal medium was added, and protoplasts pelleted by centrifugation. The pellet was washed by resuspension and re-centrifugation with 1ml 20% sucrose in Aspergillus minimal medium and resuspended in 1ml of the same solution. This was added to 9ml 20% sucrose in Aspergillus minimal medium with 1% low-melt agar held at 42°C, and after mixing gently, poured into a petri dish. Plates were incubated at 27°C for 24h prior to addition of 4ml 300ug/ml chlorimuron ethyl in Aspergillus minimal medium with 1% low melt agarose. Plates were further incubated until colonies of transformants were observed, and these were individually transferred to yeast mannitol agar plates for subsequent culture and colony purification. The resulting transformed *Metarhizium* strain 15013-1 expressing ZsGreen was designated as "Met 26."

### Example 2. Conventional microsclerotia preparation (not part of the claimed invention)

Conidiospores were collected from sporulating cultures of the Met 26 strain growing on potato dextrose agar (PDA) plates at 28°C, into 0.05% Tween 80, using a sterile spreader. Spore suspensions were filtered through 70µm Nylon mesh, to remove hyphal material, and counted using a hemocytometer. Suspensions were brought to 0.4 × 10⁸ conidia mL⁻¹ with water, and 12.5ml used to inoculate baffled 250ml flasks containing microsclerotia production medium. After eight days at 28°C, 300rpm, the cultures were harvested by filtration under low vacuum on a 150um mesh sieve. After washing with sterile water, the microsclerotia were scraped off the sieve and resuspended to 25% (w/v) in sterile water. Celite 545 was added to 25% (w/v), and after thorough mixing, the preparation was dewatered under vacuum on a 45um filter. The dewatered material was transferred to a biohood and allowed to dry for 24h in the biohood airflow. The resultant dried powder was stored under vacuum at 15°C.

Viability of microsclerotia was tested by resuspending to 1.0 mg/ml in 0.05% Tween 20 solution, and spreading 0.1ml evenly onto water agar plates. After 7 days at 28°C, plates were imaged using a FUJI LAS4000 instrument and growing fluorescent colonies counted. This analysis indicated that an average of 2600 colonies (germinating microsclerotia) were present per mg of dried material. Conidiospores were collected from these plates into 0.05% Tween solution using a spreader, and counted using a hemocytometer. This analysis indicated that an average of 6.66 × 10⁶ conidiospores were produced per mg of dried material.

### Example 3. Stability of conventional microsclerotia preparations (comparative example)

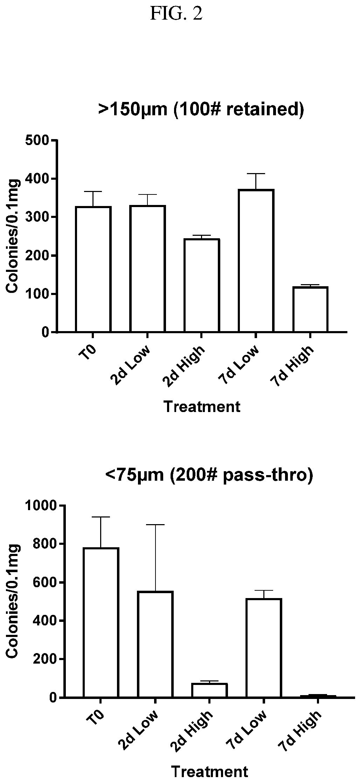

Samples of conventionally-produced microsclerotia were placed in sealed vessels over either saturated NaOH or CaNO₃ (low and high humidity, respectively - about 6 and 55%), and material withdrawn at different times subsequently for viability testing. Viability was tested by resuspending the solid material in 0.05% Tween solution to 1mg/ml, and spreading 100µl of this suspension over the surface of a water agar plate. After 10d at 28°C, growing fluorescent colonies were imaged and colonies counted using a LAS4000 instrument (Fuji). The results for colony forming units (CFU) are shown in Table 1. The viability of samples was measured at the beginning of the experiment and following 2 and 7 days storage. Both preparations lost activity during storage, particularly under conditions of high humidity, and the 'Size reduced' fraction was most affected, losing 99% viability after 7d under high humidity (See FIG. 2). These results showed that damaged or fragmented microsclerotia are less stable than larger, more intact microsclerotia. The larger microsclerotia (>150µm) also suffered significant loss of viability on storage, however, 74% viable after 2d, and only 36% viable after one week. Storage of this material under low humidity conditions resulted in 100% retention of viability.

**Table 1. Viability of conventionally prepared microsclerotia**

| **Sampling time** | **Fraction** | **Colonies/0.1mg** | | **Average** | | **% viability** | | **% loss of viability** | |
|---|---|---|---|---|---|---|---|---|---|
| | | | | | | | | | |
| Day 0 | 150µm retained | | 367 | | 328 | | 100 | | - |
| | | | 290 | | | | | | |
| | 74µm pass-through | | 624 | | 782 | | 100 | | - |
| | | | 940 | | | | | | |
| Day 2 | 150µm retained; high humidity | | 237 | | 242 | | 74 | | 26 |
| | | | 248 | | | | | | |
| | 150µm retained; low humidity | | 359 | | 330 | | 100 | | 0 |
| | | | 301 | | | | | | |
| | 75µm pass-through; high humidity | | 67 | | 77 | | 10 | | 90 |
| | | | 88 | | | | | | |
| | 75µm pass-through; low humidity | | 900 | | 556 | | 71 | | 29 |
| | | | 213 | | | | | | |
| Day 7 | 150µm retained; high humidity | | 125 | | 119 | | 36 | | 64 |
| | | | 117 | | | | | | |
| | | | 116 | | | | | | |
| | 150µm retained; low humidity | | 357 | | 372 | | 100 | | 0 |
| | | | 419 | | | | | | |
| | | | 342 | | | | | | |
| | 75µm pass-through; high humidity | | 16 | | 14 | | 1 | | 99 |
| | | | 12 | | | | | | |
| | | | 14 | | | | | | |
| | 75µm pass-through; low humidity | | 535 | | 518 | | 66 | | 34 |
| | | | 549 | | | | | | |
| | | | 471 | | | | | | |
| | | | | | | | | | |

| | | | | | | | | | |
|---|---|---|---|---|---|---|---|---|---|
| *Rt means room temperature | | | | | | | | | |

### Example 4. Production of microsclerotia for alginate encapsulation (not part of the claimed invention)

Conidiospores were collected from sporulating cultures of the Met 26 strain growing on potato dextrose agar (PDA) plates at 28°C, into 0.05% Tween 80, using a sterile spreader. Spore suspensions were filtered through 70µm Nylon mesh, to remove hyphal material, and counted using a hemocytometer. Suspensions were brought to 0.4 × 10⁸ conidia mL⁻¹ with water, and 12.5ml used to inoculate baffled 250ml flasks containing microsclerotia production medium. After eight days at 28°C, 300rpm, the cultures were harvested by filtration under low vacuum on a 150um mesh sieve. After washing with sterile water, the microsclerotia were scraped off the sieve and resuspended to 25% (w/v) in sterile water.

### Example 5. Microsclerotia Production Medium (not part of the claimed invention)

The following was added to 250ml baffled Erlenmeyer flasks:
- 50ml 2x concentrate of basal medium

Autoclave, then add immediately prior to inoculation:
- 37.5ml 20% glucose

| Basal Medium | | |
|---|---|---|
| Per L: | | 2x |
| Yeast extract | 12.5g | 25g |
| KH2PO4 | 2.0 g | 4g |
| CaCl2 · 2H2O | 0.4 g | 0.8g |
| MgSO4 · 7H2O | 0.3 g | 0.6g |
| FeSO4 · 7H2O | 0.05 g | 0.1g |
| CoCl2 · 6H2O | 37 mg | 20ml 100x stock |
| MnSO4 · H2O | 16 mg | 20ml 100x stock |
| ZnSO4 · 7H2O | 14 mg | 20ml 100x stock |
| Cobalt chloride stock solution (100×): | | |
| 1.83 g CoCl 2·6H2O in 500 mL deionized water. | | |
| Manganese sulfate stock solution (100×): | | |
| 0.78 g MnSO·4H2O in 500 mL deionized water. | | |
| Zinc sulfate stock solution (100×): | | |
| 0.70 g ZnSO 4·7H2O in 500 mL deionized water. | | |

### Example 6. Encapsulation of microsclerotia into alginate beads

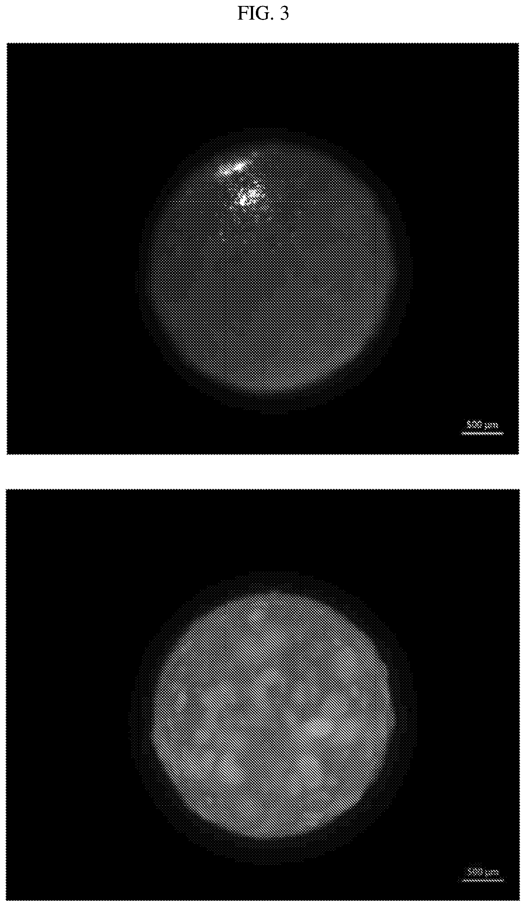

One part Met26 microsclerotia (25% w/v) was mixed with two parts 3% (w/v) sodium alginate solution. The mixture was expelled slowly into a stirred solution of 0.1M CaCl₂ through a 18G needle. The resultant beads were allowed to harden for 20min, then excess CaCl₂ solution was poured off and beads collected on an 850um mesh sieve. Beads were washed on the filter with sterile water, then suspended in sterile water for 20min with stirring. Beads were then collected onto an 850um mesh sieve. Beads were dried by exposure to the airflow of a biohood for 24h. FIG. 3 shows an example of Met 26 microsclerotia encapsulated into a bead prior to drying.

### Example 7. Viability of encapsulated microsclerotia

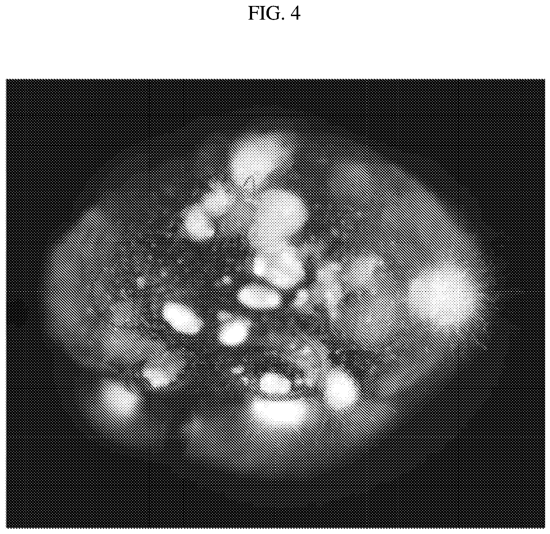

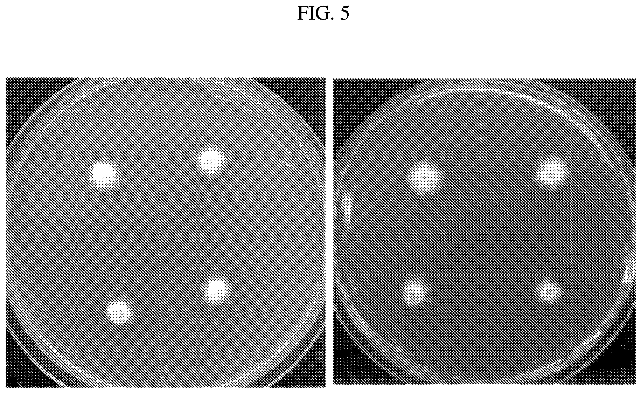

Beads, either freshly prepared or dried, were inserted into PDA or water agar in petri dishes and incubated at 28°C. Germination of microsclerotia and hyphal emergence was observed by fluorescence microscopy initially, until growing cultures could be observed with the human eye (See FIG. 4). 100% of beads tested, dried or undried, produced colonies of *Metarhizium* within days of plating. A micrograph showing microsclerotia germination and hyphal emergence from a dried bead on water agar is shown in FIG. 5.

### Example 8. Storage stability of encapsulated microsclerotia

Samples of conventionally-produced (unencapsulated) and alginate-encapsulated microsclerotia were placed in closed vessels over either saturated NaOH or CaNO₃ (low and high humidity, respectively - about 6 and 55%), held at a constant temperature of 20°C. Samples were taken initially and at weekly intervals thereafter for viability testing. Viability of microsclerotia was tested by resuspending to 10mg/ml in 0.05% Tween 20 solution, and spreading 0.1ml evenly onto water agar plates. After 10 days at 28°C, conidiospores were collected into 0.05% Tween solution using a spreader, and counted using a hemocytometer. Viability of encapsulated microsclerotia was tested by pressing individual beads into the surface of 1ml water agar plugs in a 24-well plate. After 10 days at 28°C, agar plugs were transferred to tubes containing 1ml 0.05% Tween solution and conidiospores suspended by vortex mixing (15s). Conidiospores were counted using a hemocytometer.

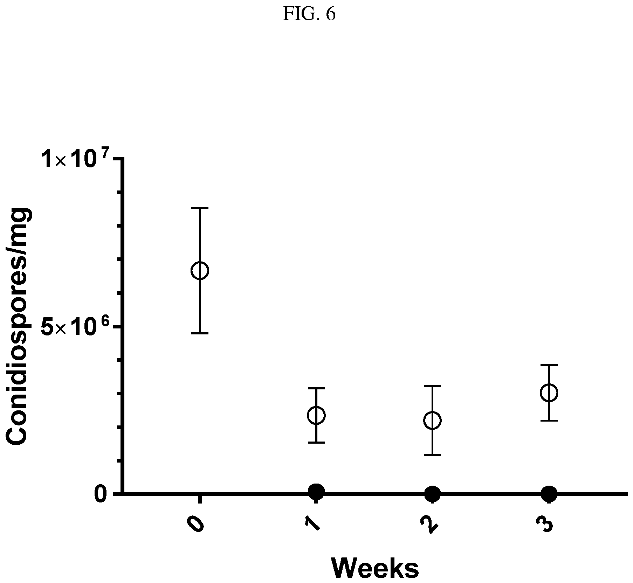

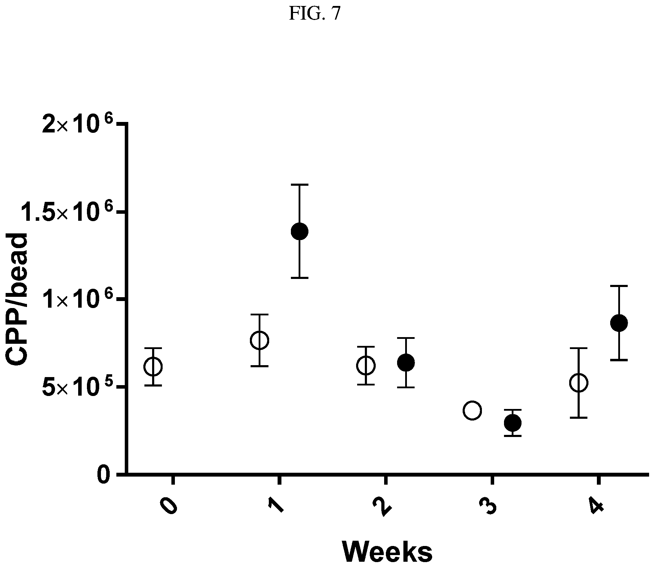

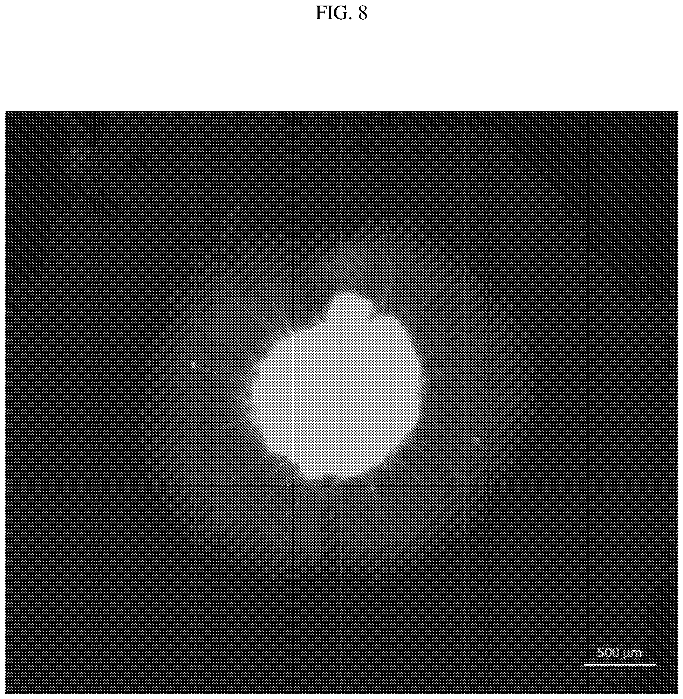

Storage of unencapsulated microsclerotia under high humidity conditions resulted in a rapid loss of viability, with no detectable conidiospore production after one week (FIG. 6). Encapsulated microsclerotia stored under the same conditions retained viability for four weeks (FIG. 7). After four weeks at 20°C, the mean conidiospore productivity per bead of encapsulated microscleotia, at high or low humidity, were statistically identical. Beads held at low humidity produced 0.52 +/- 0.19 × 10⁶ spores, with beads held at high humidity producing 0.86 +/- 0.21 × 10⁶ spores. FIG. 8 shows hyphal emergence from a dried alginate bead in water agar, after one week of storage at high humidity.

### Example 9. Establishment in soil of Metarhizium encapsulated into dried alginate beads

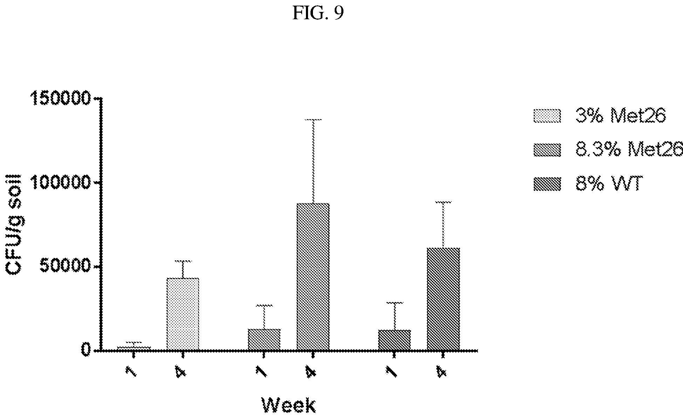

Alginate beads containing *Metarhizium* Met26 (3 and 8.3%, w/v) and 15013-1 (8% w/v) were prepared and dried. A single dried bead incorporating *Metarhizium* Met26 or wild-type 15013-1 microsclerotia was sown 1cm below the surface of 20g plugs of wet topsoil: sand (1:1 by volume) in a plug tray. The plug tray was covered and incubated at 28°C, with water added periodically to maintain a moist condition. The soil was sampled after one and four weeks incubation. Samples consisted the entirety of soil from individual plugs (about 20g). Samples were transferred to 50ml sterile tubes, and 20ml 0.05% Tween 80 added. The tubes were vortex mixed, then soil allowed to settle. The supernatant was diluted 20x in sterile water, and 0.1ml spread evenly onto Rose Bengal Agar plates containing chloramphenicol and thiabendazole (15uM). Plates were incubated at 28°C for five days. Fluorescent colonies of *Metarhizium* Met26 were observed and counted using a FUJI LAS4000 imaging system. Non-fluorescent 15013-1 colonies were enumerated by observation. The results (FIG. 9) show that *Metarhizium* microsclerotia incorporated within dried alginate beads established and proliferated after placing into soil.

### Example 10. Stability of Metarhizium microsclerotia incorporated into dried alginate microbeads

One part Met26 microsclerotia (12% w/v) was mixed with two parts 3% (w/v) sodium alginate solution. The mixture was pumped onto a spinning disk atomizer (Ransburg Turbodisk^{™} 6in serrated disk, not anodized, PIN 20485-62) at ca. 1L/h through a 18G needle, with disk speed 1000rpm. Drops were collected into a solution of 0.1M CaCl2. The resultant beads were allowed to harden for 15min, then beads collected on a 250um mesh sieve. Beads were washed on the sieve with sterile water, then suspended in sterile water for 15min with stirring. Beads were then collected on a 250um mesh sieve. Beads were allowed to dry overnight in the sterile airflow of a biohood and stored under vacuum at 15°C. Microscopic analysis of the dried beads indicated an average maximum dimension of 354um.

Viability of microencapsulated microsclerotia was tested by suspending to 10mg/ml in Tween 20 solution and spreading 0.1ml onto water agar plates. After 7 days at 28°C, conidiospores were suspended in 0.05% Tween 20 solution using a spreader and counted using a hemocytometer. This analysis indicated the microcapsules had a conidiospore production potential (CPP) of 4.81 × 10⁶ per mg.

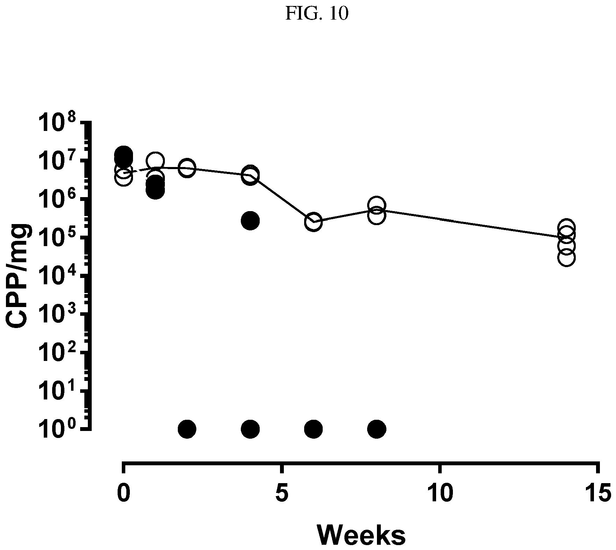

A sample of the microbeads was placed in a constant environment chamber (Percival) held at 25°C and 55% humidity. A sample of conventionally-prepared, unencapsulated microsclerotia (CPP of 12.68 × 10⁶/mg) was similarly treated. At intervals thereafter, samples were removed for viability analysis. Viability of encapsulated and unencapsulated microsclerotia was tested by resuspending to 10mg/ml in 0.05% Tween 20 solution, and spreading 0.1ml evenly onto water agar plates. After 10 days at 28°C, conidiospores were collected into 0.05% Tween solution using a spreader, and counted using a hemocytometer. Encapsulation resulted in preservation of viability over the duration of the experiment, with 0.69 log reduction in CPP of encapsulated at 14 weeks compared to the complete loss of viability of the unencapsulated material after four weeks (See FIG. 10).

## Claims

1. A method of preparing fungal entomopathogenic microsclerotia composition from a culture of microsclerotia comprising:
a) mixing the microsclerotia with an alginate salt solution to form a mixture of the microsclerotia and the alginate salt solution; and
b) adding the mixture to a multivalent cation solution to form an alginate bead encapsulating the microsclerotia;
wherein the microsclerotia in the alginate bead have enhanced stability;
and wherein the fungal entomopathogenic microsclerotia comprises at least one strain selected from the group consisting of:
(i) the fungal entomopathogen deposited as Patent Deposit No. NRRL 67073;
(ii) the fungal entomopathogen deposited as Patent Deposit No. NRRL 67074; and
(iii) the fungal entomopathogen deposited as Patent Deposit No. NRRL 67075.

2. The method of claim 1:
(a) further comprising collecting the microsclerotia from the culture prior to mixing with the alginate salt solution; preferably wherein the collecting is performed by filtration or centrifugation;
(b) wherein the multivalent cation solution comprises a salt of calcium, wherein the salt of calcium further comprises carbonate, chloride, lactate, or gluconate;
(c) further comprising drying the alginate beads encapsulating the microsclerotia;
(d) further comprising breaking the alginate beads encapsulating the microsclerotia into smaller bead fragments;
(e) further comprising coating a seed with the alginate beads encapsulating the microsclerotia, or with fragments thereof;
(f) further comprising planting the alginate beads encapsulating the microsclerotia infurrow with a seed;
(g) further comprising applying the alginate beads encapsulating the microsclerotia to the soil proximate to a plant or plant part;
(h) further comprising applying the alginate beads encapsulating the microsclerotia to the soil prior to or after planting a seed;
(i) further comprising applying the alginate beads encapsulating the microsclerotia to a plant or plant part;
(j) wherein the alginate beads encapsulating the microsclerotia increases resistance to a plant pathogen, pest or insect;
(k) wherein the microsclerotia in the alginate bead have increased stability, increased germination, or increased establishment in the soil compared to microsclerotia not encapsulated in an alginate bead; or
(l) wherein the enhanced stability comprises viability of the microsclerotia for at least 2 weeks after encapsulation.

3. A composition comprising at least one entomopathogenic fungal strain encapsulated in an alginate bead; wherein the entomopathogenic fungal strain is selected from the group consisting of:
(i) the fungal entomopathogen deposited as Patent Deposit No. NRRL 67073;
(ii) the fungal entomopathogen deposited as Patent Deposit No. NRRL 67074; and
(iii) the fungal entomopathogen deposited as Patent Deposit No. NRRL 67075.

4. The composition of claim 3:
(a) wherein the composition further comprises a biocontrol agent or plant growth-promoting agent selected from the group consisting of bacteria, a fungus, a yeast, protozoa, a virus, an entomopathogenic nematode, a botanical extract, a protein, a nucleic acid, a secondary metabolite, and an inoculant;
(b) wherein the composition further comprises a compound selected from the group consisting of a safener, a lipo-chitooligosaccharide, a benzoxazinoid, a triglucosamine lipoglycine salt, an isoflavone, and a ryanodine receptor modulator; or
(c) further comprising a plant or plant part; preferably wherein the plant or the plant part is genetically modified or transgenic; more preferably wherein the genetically modified plant part is a seed or a leaf.

5. The composition of Claim 3, wherein the composition further comprises an agrochemically active compound selected from the group consisting of an insecticide, a fungicide, a bactericide, and a nematicide.

6. The composition of Claim 5, wherein the agrochemically active compound is a fungicide;
preferably wherein the fungicide comprises a fungicide composition selected from the group consisting of azoxystrobin, thiabendazole, fludioxonil, metalaxyl, tebuconazole, prothioconazole, ipconazole, penflufen, and sedaxane.

7. A composition comprising at least one microsclerotium of at least one entomopathological fungal strain encapsulated in an alginate bead; wherein the entomopathogenic fungal strain is selected from the group consisting of:
(i) the fungal entomopathogen deposited as Patent Deposit No. NRRL 67073;
(ii) the fungal entomopathogen deposited as Patent Deposit No. NRRL 67074; and
(iii) the fungal entomopathogen deposited as Patent Deposit No. NRRL 67075.

8. The composition of claim 7, wherein:
(b) the composition further comprises a biocontrol agent or plant growth-promoting agent selected from the group consisting of bacteria, a fungus, a yeast, protozoa, a virus, an entomopathogenic nematode, a botanical extract, a protein, a nucleic acid, a secondary metabolite, and an inoculant; or
(c) the composition further comprises a compound selected from the group consisting of a safener, a lipo-chitooligosaccharide, a benzoxazinoid, a triglucosamine lipoglycine salt, an isoflavone, and a ryanodine receptor modulator.

9. The composition of claim 7, wherein the composition further comprises an agrochemically active compound selected from the group consisting of an insecticide, a fungicide, a bactericide, and a nematicide.

10. The composition of claim 9, wherein the agrochemically active compound is a fungicide.

11. The composition of claim 10, wherein the fungicide comprises a fungicide composition selected from the group consisting of azoxystrobin, thiabendazole, fludioxonil, metalaxyl, tebuconazole, prothioconazole, ipconazole, penflufen, and sedaxane.

12. The composition of claim 7, further comprising a plant or plant part.

13. The composition of claim 12, wherein the plant or the plant part is genetically modified or transgenic.

14. The composition of Claim 13, wherein the genetically modified plant part is a seed or a leaf.

## Patentansprüche

1. Verfahren zur Herstellung einer pilzlichen entomopathogenen Mikrosklerotien-Zusammensetzung aus einer Kultur von Mikrosklerotien, umfassend:
a) das Mischen der Mikrosklerotien mit einer Alginatsalzlösung unter Bildung einer Mischung aus den Mikrosklerotien und der Alginatsalzlösung und
b) die Zugabe der Mischung zu einer Lösung mehrwertiger Kationen unter Bildung eines die Mikrosklerotien einkapselnden Alginatkügelchens,
wobei die Mikrosklerotien in dem Alginatkügelchen eine erhöhte Stabilität aufweisen
und wobei die pilzlichen entomopathogenen Mikrosklerotien mindestens einen aus der aus:
(i) dem als Patent Deposit No. NRRL 67073 hinterlegten pilzlichen Entomopathogen,
(ii) dem als Patent Deposit No. NRRL 67074 hinterlegten pilzlichen Entomopathogen und
(iii) dem als Patent Deposit No. NRRL 67075 hinterlegten pilzlichen Entomopathogen bestehenden Gruppe ausgewählten Stamm umfassen.

2. Verfahren nach Anspruch 1:
(a) weiterhin umfassend das Isolieren der Mikrosklerotien aus der Kultur vor dem Mischen mit der Alginatsalzlösung, wobei das Isolieren vorzugsweise durch Filtrieren oder Zentrifugieren erfolgt,
(b) wobei die Lösung der mehrwertigen Kationen ein Calciumsalz umfasst, wobei das Calciumsalz weiterhin Carbonat, Chlorid, Lactat oder Gluconat umfasst,
(c) weiterhin umfassend das Trocknen der die Mikrosklerotien einkapselnden Alginatkügelchen,
(d) weiterhin umfassend das Aufbrechen der die Mikrosklerotien einkapselnden Alginatkügelchen zu kleineren Kügelchenfragmenten,
(e) weiterhin umfassend das Beschichten eines Samens mit den die Mikrosklerotien einkapselnden Alginatkügelchen oder mit Fragmenten davon,
(f) weiterhin umfassend das Einpflanzen der die Mikrosklerotien einkapselnden Alginatkügelchen in der Furche mit einem Samen,
(g) weiterhin umfassend das Einbringen der die Mikrosklerotien einkapselnden Alginatkügelchen in den Boden in der Nähe einer Pflanze oder eines Pflanzenteils,
(h) weiterhin umfassend das Einbringen der die Mikrosklerotien einkapselnden Alginatkügelchen in den Boden vor oder nach dem Einpflanzen eines Samens,
(i) weiterhin umfassend das Aufbringen der die Mikrosklerotien einkapselnden Alginatkügelchen auf eine Pflanze oder einen Pflanzenteil,
(j) wherein die die Mikrosklerotien einkapselnden Alginatkügelchen die Resistenz gegenüber einem Pflanzenpathogen, einem Schädling oder einem Insekt erhöhen,
(k) wobei die Mikrosklerotien in dem Alginatkügelchen verbesserte Stabilität, verbesserte Keimung oder verbesserte Etablierung im Boden im Vergleich zu nicht in einem Alginatkügelchen eingekapselten Mikrosklerotien aufweisen oder
(l) wobei die verbesserte Stabilität eine mindestens 2-wöchige Lebensfähigkeit der Mikrosklerotien nach der Einkapselung umfasst.

3. Zusammensetzung, umfassend mindestens einen entomopathogenen pilzlichen Stamm eingekapselt in einem Alginatkügelchen, wobei der entomopathogene pilzliche Stamm aus der aus:
(i) dem als Patent Deposit No. NRRL 67073 hinterlegten pilzlichen Entomopathogen,
(ii) dem als Patent Deposit No. NRRL 67074 hinterlegten pilzlichen Entomopathogen und
(iii) dem als Patent Deposit No. NRRL 67075 hinterlegten pilzlichen Entomopathogen bestehenden Gruppe ausgewählt ist.

4. Zusammensetzung nach Anspruch 3:
(a) wobei die Zusammensetzung weiterhin ein biologisches Bekämpfungsmittel oder ein das Pflanzenwachstum förderndes Mittel ausgewählt aus der aus Bakterien, einem Pilz, einer Hefe, Protozoen, einem Virus, einer entomopathogenen Nematode, einem botanischen Extrakt, einem Protein, einer Nukleinsäure, einem sekundären Metaboliten und einem Inokulum bestehenden Gruppe umfasst,
(b) wobei die Zusammensetzung weiterhin eine aus der aus einem Safener, einem Lipochitooligosaccharid, einem Benzoxazinoid, einem Triglucosaminlipoglycinsalz, einem Isoflavon und einem Ryanodinrezeptormodulator bestehenden Gruppe ausgewählte Verbindung umfasst oder
(c) weiterhin umfassend eine Pflanze oder einem Pflanzenteil, wobei die Pflanze bzw. der Pflanzenteil vorzugsweise genetisch modifiziert oder transgen ist, wobei es sich bei dem genetisch modifizierten Pflanzenteil besonders bevorzugt um einen Samen oder ein Blatt handelt.

5. Zusammensetzung nach Anspruch 3, wobei die Zusammensetzung weiterhin einen aus der aus einem Insektizid, einem Fungizid, einem Bakterizid und einem Nematizid bestehenden Gruppe ausgewählten agrochemischen Wirkstoff umfasst.

6. Zusammensetzung nach Anspruch 5, wobei es sich bei dem agrochemischen Stoff um ein Fungizid handelt, wobei das Fungizid vorzugsweise eine aus der aus Azoxystrobin, Thiabendazol, Fludioxonil, Metalaxyl, Tebuconazol, Prothioconazol, Ipconazol, Penflufen und Sedaxan bestehenden Gruppe ausgewählte Fungizidzusammensetzung umfasst.

7. Zusammensetzung, umfassend mindestens ein Mikrosklerotium mindestens eines entomopathologischen pilzlichen Stamms eingekapselt in einem Alginatkügelchen, wobei der entomopathogene pilzliche Stamm aus der aus:
(i) dem als Patent Deposit No. NRRL 67073 hinterlegten pilzlichen Entomopathogen,
(ii) dem als Patent Deposit No. NRRL 67074 hinterlegten pilzlichen Entomopathogen und
(iii) dem als Patent Deposit No. NRRL 67075 hinterlegten pilzlichen Entomopathogen bestehenden Gruppe ausgewählt ist.

8. Zusammensetzung nach Anspruch 7, wobei:
(b) die Zusammensetzung weiterhin ein biologisches Bekämpfungsmittel oder ein das Pflanzenwachstum förderndes Mittel ausgewählt aus der aus Bakterien, einem Pilz, einer Hefe, Protozoen, einem Virus, einer entomopathogenen Nematode, einem botanischen Extrakt, einem Protein, einer Nukleinsäure, einem sekundären Metaboliten und einem Inokulum bestehenden Gruppe umfasst oder
(c) die Zusammensetzung weiterhin eine aus der aus einem Safener, einem Lipochitooligosaccharid, einem Benzoxazinoid, einem Triglucosaminlipoglycinsalz, einem Isoflavon und einem Ryanodinrezeptormodulator bestehenden Gruppe ausgewählte Verbindung umfasst.

9. Zusammensetzung nach Anspruch 7, wobei die Zusammensetzung weiterhin einen aus der aus einem Insektizid, einem Fungizid, einem Bakterizid und einem Nematizid bestehenden Gruppe ausgewählten agrochemischen Wirkstoff umfasst.

10. Zusammensetzung nach Anspruch 9, wobei es sich bei dem agrochemischen Wirkstoff um ein Fungizid handelt.

11. Zusammensetzung nach Anspruch 10, wobei das Fungizid eine aus der aus Azoxystrobin, Thiabendazol, Fludioxonil, Metalaxyl, Tebuconazol, Prothioconazol, Ipconazol, Penflufen und Sedaxan bestehenden Gruppe ausgewählte Fungizidzusammensetzung umfasst.

12. Zusammensetzung nach Anspruch 7, weiterhin umfassend eine Pflanze oder einen Pflanzenteil.

13. Zusammensetzung nach Anspruch 12, wobei die Pflanze oder der Pflanzenteil genetisch modifiziert oder transgen ist.

14. Zusammensetzung nach Anspruch 13, wobei es sich bei dem genetisch modifizierten Pflanzenteil um einen Samen oder ein Blatt handelt.

## Revendications

1. Procédé de préparation d'une composition de microsclérotes entomopathogènes fongiques à partir d'une culture de microsclérotes comprenant :
a) le mélange des microsclérotes avec une solution de sel d'alginate pour former un mélange des microsclérotes et de la solution de sel d'alginate ; et
b) l'ajout du mélange à une solution de cations multivalents pour former une bille d'alginate encapsulant les microsclérotes ;
les microsclérotes dans la bille d'alginate ayant une stabilité améliorée ;
et les microsclérotes entomopathogènes fongiques comprenant au moins une souche choisie dans le groupe constitué par :
(i) l'entomopathogène fongique déposé sous le numéro de dépôt de brevet NRRL 67073 ;
(ii) l'entomopathogène fongique déposé sous le numéro de dépôt de brevet NRRL 67074 ; et
(iii) l'entomopathogène fongique déposé sous le numéro de dépôt de brevet NRRL 67075.

2. Procédé selon la revendication 1 :
(a) comprenant en outre la collecte des microsclérotes de la culture avant le mélange avec la solution de sel d'alginate ; préférablement la collecte étant réalisée par filtration ou centrifugation ;
(b) la solution de cations multivalents comprenant un sel de calcium, le sel de calcium comprenant en outre carbonate, chlorure, lactate ou gluconate ;
(c) comprenant en outre le séchage des billes d'alginate encapsulant les microsclérotes ;
(d) comprenant en outre la rupture des billes d'alginate encapsulant les microsclérotes en fragments de billes plus petits ;
(e) comprenant en outre le revêtement d'une graine par les billes d'alginate encapsulant les microsclérotes, ou par des fragments correspondants ;
(f) comprenant en outre la plantation des billes d'alginate encapsulant les microsclérotes dans le sillon avec une graine ;
(g) comprenant en outre l'application des billes d'alginate encapsulant les microsclérotes sur le sol à proximité d'un végétal ou d'une partie de végétal ;
(h) comprenant en outre l'application des billes d'alginate encapsulant les microsclérotes sur le sol avant ou après la plantation d'une graine ;
(i) comprenant en outre l'application des billes d'alginate encapsulant les microsclérotes sur un végétal ou une partie de végétal ;
(j) les billes d'alginate encapsulant les microsclérotes augmentant la résistance à un pathogène végétal, un organisme nuisible ou un insecte ;
(k) les microsclérotes dans la bille d'alginate ayant une stabilité augmentée, une germination augmentée ou un établissement augmenté dans le sol par comparaison avec des microsclérotes non encapsulés dans une bille d'alginate ; ou
(l) la stabilité améliorée comprenant la viabilité des microsclérotes pendant au moins 2 semaines après encapsulation.

3. Composition comprenant au moins une souche fongique entomopathogène encapsulée dans une bille d'alginate ; la souche fongique entomopathogène étant choisie dans le groupe constitué par :
(i) l'entomopathogène fongique déposé sous le numéro de dépôt de brevet NRRL 67073 ;
(ii) l'entomopathogène fongique déposé sous le numéro de dépôt de brevet NRRL 67074 ; et
(iii) l'entomopathogène fongique déposé sous le numéro de dépôt de brevet NRRL 67075.

4. Composition selon la revendication 3 :
(a) la composition comprenant en outre un agent de lutte biologique ou un agent de promotion de la croissance végétale choisi dans le groupe constitué par des bactéries, un champignon, une levure, des protozoaires, un virus, un nématode entomopathogène, un extrait botanique, une protéine, un acide nucléique, un métabolite secondaire, et un inoculant ;
(b) la composition comprenant en outre un composé choisi dans le groupe constitué par un agent phytoprotecteur, un lipo-chitooligosaccharide, un benzoxazinoïde, un sel de lipoglycine de triglucosamine, une isoflavone et un modulateur des récepteurs de la ryanodine ; ou
(c) comprenant en outre un végétal ou une partie de végétal, préférablement le végétal ou la partie de végétal étant génétiquement modifié(e) ou transgénique ; plus préférablement la partie de végétal génétiquement modifiée étant une graine ou une feuille.

5. Composition selon la revendication 3, la composition comprenant en outre un composé actif sur le plan agrochimique choisi dans le groupe constitué par un insecticide, un fongicide, un bactéricide et un nématicide.

6. Composition selon la revendication 5, le composé actif sur le plan agrochimique étant un fongicide ; préférablement le fongicide comprenant une composition de fongicide choisie dans le groupe constitué par l'azoxystrobine, le thiabendazole, le fludioxonil, le métalaxyl, le tébuconazole, le prothioconazole, l'ipconazole, le penflufène et le sédaxane.

7. Composition comprenant au moins un microsclérote d'au moins une souche fongique entomopathogène encapsulée dans une bille d'alginate ; la souche fongique entomopathogène étant choisie dans le groupe constitué par :
(i) l'entomopathogène fongique déposé sous le numéro de dépôt de brevet NRRL 67073 ;
(ii) l'entomopathogène fongique déposé sous le numéro de dépôt de brevet NRRL 67074 ; et
(iii) l'entomopathogène fongique déposé sous le numéro de dépôt de brevet NRRL 67075.

8. Composition selon la revendication 7,
(b) la composition comprenant en outre un agent de lutte biologique ou un agent de promotion de la croissance végétale choisi dans le groupe constitué par des bactéries, un champignon, une levure, des protozoaires, un virus, un nématode entomopathogène, un extrait botanique, une protéine, un acide nucléique, un métabolite secondaire, et un inoculant ; ou
(c) la composition comprenant en outre un composé choisi dans le groupe constitué par un agent phytoprotecteur, un lipo-chitooligosaccharide, un benzoxazinoïde, un sel de lipoglycine de triglucosamine, une isoflavone et un modulateur des récepteurs de la ryanodine.

9. Composition selon la revendication 7, la composition comprenant en outre un composé actif sur le plan agrochimique choisi dans le groupe constitué par un insecticide, un fongicide, un bactéricide et un nématicide.

10. Composition selon la revendication 9, le composé actif sur le plan agrochimique étant un fongicide.

11. Composition selon la revendication 10, le fongicide comprenant une composition de fongicide choisie dans le groupe constitué par l'azoxystrobine, le thiabendazole, le fludioxonil, le métalaxyl, le tébuconazole, le prothioconazole, l'ipconazole, le penflufène et le sédaxane.

12. Composition selon la revendication 7, comprenant en outre un végétal ou une partie de végétal.

13. Composition selon la revendication 12, le végétal ou la partie de végétal étant génétiquement modifié(e) ou transgénique.

14. Composition selon la revendication 13, la partie de végétal génétiquement modifiée étant une graine ou une feuille.
